# EUROPEAN PATENT SPECIFICATION

(11) **EP 0 602 126 B1**
(45) Date of publication and mention of the grant of the patent: **05.03.2003**
(21) Application number: 92918713.6
(22) Date of filing: 14.08.1992
(51) Int. Cl.: C12N 15/13, C07K 16/18, C07K 16/42, A61K 39/395

(54) **IMMUNOGLOBULIN VARIANTS FOR SPECIFIC FC EPSILON RECEPTORS**
Veränderte Immunglobuline für spezifische FC-Epsilon Rezeptoren
VARIANTES D'IMMUNOGLOBULINES POUR DES RECEPTEURS SPECIFIQUES FC EPSILON

(30) Priority: 14.08.1991 US 744768; 07.05.1992 US 879495
(43) Date of publication of application: 22.06.1994
(60) Divisional application: 02009009.8
(73) Proprietor: GENENTECH, INC., South San Francisco California 94080 (US)
(72) Inventor: JARDIEU, Paula, M., Berkeley, CA 94708 (US); PRESTA, Leonard, G., San Francisco, CA 94109 (US)
(74) Representative: Armitage, Ian Michael
(86) International application number: US9206860
(87) International publication number: WO93004173

(56) References cited:
- JOURNAL OF BIOLOGICAL CHEMISTRY. vol. 266, no. 17, 15 June 1991, BALTIMORE US pages 11245 - 11251 RISKE, F. ET AL. 'High affinity human IgE receptor (FcERI)
- NATURE. vol. 338, 20 April 1989, LONDON GB pages 649 - 651 VERCELLI, D. ET AL. 'The B-cell binding site on human immunoglobulin E.'
- PROCEEDINGS OF THE NATIONAL ACADEMY OF SCIENCES OF USA. vol. 81, September 1984, WASHINGTON US pages 5369 - 5373 LIU, F.T. ET AL. 'Expression of a biologically active fragment of human IgE epsilon chain in Escherichia coli.'
- NUCLEIC ACIDS RESEARCH. vol. 11, no. 10, 1983, ARLINGTON, VIRGINIA US pages 3077 - 3085 KUROKAWA, T. ET AL. 'Expression of human immunoglobulin epsilon chain cDNA in E coli'
- EUROPEAN JOURNAL OF IMMUNOLOGY vol. 17, 1987, VCH VERLAGSGESELLSCHAFT, DEUTSCHLAND pages 437 - 440 BURT, D.S. ET AL. 'Inhibition of binding of rat IgE to rat mast cells by synthetic IgE peptides.'
- THE JOURNAL OF ALLERGY AND CLINICAL IMMUNOLOGY vol. 79, no. 1, 1987, PAGE 20,ABSTRACT 20; GEHA, R.S. ET AL. 'IgE sites relevant for binding to type 1 Fc epsilon (FCER) receptors on mast cells.
- FASEB JOURNAL. vol. 2, no. 1, January 1988, BETHESDA, MD US pages 14 - 17 KINET, J.P. ET AL. 'How antibodies work : focus on Fc receptors'
- PRESTA ET AL.: 'Humanization of an antibody directed against IgE' J. IMMUNOL. vol. 151, no. 5, 01 September 1993, pages 2623 - 2632

### SEQUENCE LISTING

(1) GENERAL INFORMATION:
   (i) APPLICANT:
      (A) NAME: Genentech, Inc.
      (B) STREET: 460 Point San Bruno Boulevard
      (C) CITY: South San Francisco
      (D) STATE: California
      (E) COUNTRY: USA
      (F) POSTAL CODE (ZIP): 94080
   (ii) TITLE OF INVENTION: Immunoglobulin variants for specific Fc epsilon receptors
   (iii) NUMBER OF SEQUENCES: 64
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: patin (Genentech); PatentIn Release #1.0, Version #1.25 (EPO); and WordPerfect 5.1
   (v) CURRENT APPLICATION DATA:
      APPLICATION NUMBER: EP 92918713.6
   (vi) PRIOR APPLICATION DATA:
      (A) APPLICATION NUMBER: US 07/879495
      (B) FILING DATE: 07-MAY-1992
   (vi) PRIOR APPLICATION DATA:
      (A) APPLICATION NUMBER: US 07/744768
      (B) FILING DATE: 14-AUG-1991
(2) INFORMATION FOR SEQ ID NO:1:
   (i) SEQUENCE CHARACTERISTICS:
      (A) LENGTH: 118 amino acids
      (B) TYPE: amino acid
      (D) TOPOLOGY: linear
   (ix) FEATURE:
      (B) LOCATION: 1
      (D) OTHER INFORMATION: /note= "Xaa is a spacer residue"
   (ix) FEATURE:
      (B) LOCATION: 20
      (D) OTHER INFORMATION: /note= "Xaa is a spacer residue"
   (ix) FEATURE:
      (B) LOCATION: 50
      (D) OTHER INFORMATION: /note= "Xaa is a spacer residue"
   (ix) FEATURE:
      (B) LOCATION: 53
      (D) OTHER INFORMATION: /note= "Xaa is a spacer residue"
   (ix) FEATURE:
      (B) LOCATION: 54
      (D) OTHER INFORMATION: /note= "Xaa is a spacer residue"
   (ix) FEATURE:
      (B) LOCATION: 70
      (D) OTHER INFORMATION: /note= "Xaa is a spacer residue"
   (ix) FEATURE:
      (B) LOCATION: 72
      (D) OTHER INFORMATION: /note= "Xaa is a spacer residue"
   (ix) FEATURE:
      (B) LOCATION: 73
      (D) OTHER INFORMATION: /note= "Xaa is a spacer residue"
   (ix) FEATURE:
      (B) LOCATION: 108
      (D) OTHER INFORMATION: /note= "Xaa is a spacer residue"
   (xi) SEQUENCE DESCRIPTION: SEQ ID NO:1:
   Xaa Asp Ser Asn Pro Arg Gly Val Ser Ala Tyr Leu Ser Arg Pro
    1               5                  10                  15
   Ser Pro Phe Asp Xaa Leu Phe Ile Arg Lys Ser Pro Thr Ile Thr
                   20                  25                  30
   Cys Leu Val Val Asp Leu Ala Pro Ser Lys Gly Thr Val Asn Leu
                   35                  40                  45
   Thr Trp Ser Arg Xaa Ala Ser Xaa Xaa Gly Lys Pro Val Asn His
                   50                  55                  60
   Ser Thr Arg Lys Glu Glu Lys Gln Arg Xaa Asn Xaa Xaa Gly Thr
                   65                  70                  75
   Leu Thr Val Thr Ser Thr Leu Pro Val Gly Thr Arg Asp Trp Ile
                   80                  85                  90
   Glu Gly Glu Thr Gln Cys Arg Val Thr His Pro His Leu Pro Arg
                   95                  100                 105
   Ala Leu Xaa Met Arg Ser Thr Thr Lys Thr Ser Gly Pro
                   110                 115         118
(2) INFORMATION FOR SEQ ID NO:2:
   (i) SEQUENCE CHARACTERISTICS:
      (A) LENGTH: 111 amino acids
      (B) TYPE: amino acid
      (D) TOPOLOGY: linear
   (xi) SEQUENCE DESCRIPTION: SEQ ID NO:2:
   Asp Ile Val Leu Thr Gln Ser Pro Ala Ser Leu Ala Val Ser Leu
    1               5                  10                  15
   Gly Gln Arg Ala Thr Ile Ser Cys Lys Ala Ser Gln Ser Val Asp
                   20                  25                  30
   Tyr Asp Gly Asp Ser Tyr Met Asn Trp Tyr Gln Gln Lys Pro Gly
                   35                  40                  45
   Gln Pro Pro Ile Leu Leu Ile Tyr Ala Ala Ser Tyr Leu Gly Ser
                   50                  55                  60
   Glu Ile Pro Ala Arg Phe Ser Gly Ser Gly Ser Gly Thr Asp Phe
                   65                  70                  75
   Thr Leu Asn Ile His Pro Val Glu Glu Glu Asp Ala Ala Thr Phe
                   80                  85                  90
   Tyr Cys Gln Gln Ser His Glu Asp Pro Tyr Thr Phe Gly Ala Gly
                   95                 100                 105
   Thr Lys Leu Glu Ile Lys
                  110 111
(2) INFORMATION FOR SEQ ID NO:3:
   (i) SEQUENCE CHARACTERISTICS:
      (A) LENGTH: 134 amino acids
      (B) TYPE: amino acid
      (D) TOPOLOGY: linear
   (xi) SEQUENCE DESCRIPTION: SEQ ID NO:3:
   Asp Val Gln Leu Gln Glu Ser Gly Pro Gly Leu Val Lys Pro Ser
    1               5                  10                  15
   Gln Ser Leu Ser Leu Ala Cys Ser Val Thr Gly Tyr Ser Ile Thr
                   20                  25                  30
   Ser Gly Tyr Ser Trp Asn Trp Ile Arg Gln Phe Pro Gly Asn Lys
                   35                  40                  45
   Leu Glu Trp Met Gly Ser Ile Thr Tyr Asp Gly Ser Ser Asn Tyr
                   50                  55                  60
   Asn Pro Ser Leu Lys Asn Arg Ile Ser Val Thr Arg Asp Thr Ser
                   65                  70                  75
   Gln Asn Gln Phe Phe Leu Lys Leu Asn Ser Ala Thr Ala Glu Asp
                   80                  85                  90
   Thr Ala Thr Tyr Tyr Cys Ala Arg Gly Ser His Tyr Phe Gly His
                   95                 100                 105
   Trp His Phe Ala Val Trp Gly Ala Gly Thr Thr Val Thr Val Ser
                  110                 115                 120
   Ser Ala Lys Thr Thr Pro Pro Ser Val Tyr Pro Leu Ala Arg
                  125                 130             134
(2) INFORMATION FOR SEQ ID NO:4:
   (i) SEQUENCE CHARACTERISTICS:
      (A) LENGTH: 124 amino acids
      (B) TYPE: amino acid
      (D) TOPOLOGY: linear
   (xi) SEQUENCE DESCRIPTION: SEQ ID NO:4:
   Asp Ile Val Met Thr Gln Ser Gln Lys Phe Met Ser Thr Ser Val
   1 5 10 15
   Gly Asp Arg Val Ser Val Thr Cys Lys Ala Ser Gln Asn Val Ser
   20 25 30
   Ser Asn Val Ala Trp Tyr Gln Gln Lys Pro Gly Gln Ser Pro Lys
   35 40 45
   Ala Leu Ile Tyr Ser Ala Ser Tyr Arg Tyr Ser Gly Val Pro Asp
   50 55 60
   Arg Phe Thr Gly Ser Gly Ser Gly Thr Asp Phe Thr Leu Thr Ile
   65 70 75
   Ser Asn Val Gln Ser Glu Asp Leu Ala Glu Tyr Phe Cys Gln Gln
   80 85 90
   Tyr Tyr Thr Tyr Pro Leu Tyr Thr Phe Gly Gly Gly Thr Lys Leu
   95 100 105
   Glu Ile Lys Arg Ala Asp Ala Ala Pro Thr Val Ser Ile Phe Pro
   110 115 120
   Pro Ser Thr Arg
   124
(2) INFORMATION FOR SEQ ID NO:5:
   (i) SEQUENCE CHARACTERISTICS:
      (A) LENGTH: 130 amino acids
      (B) TYPE: amino acid
      (D) TOPOLOGY: linear
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 106 amino acids
      (B) TYPE: amino acid
      (D) TOPOLOGY: linear
   (xi) SEQUENCE DESCRIPTION: SEQ ID NO:6:
   Asp Ile Gln Leu Thr Gln Ser Pro Ala Ser Leu Ala Val Ser Leu
   1               5                   10                  15
   Gly Gln Arg Ala Thr Ile Ser Cys Lys Ala Ser Gln Ser Val Asp
                   20                  25                  30
   Tyr Asp Gly Asp Ser Tyr Met Asn Trp Tyr Gln Gln Lys Pro Gly
                   35                  40                  45
   Gln Pro Pro Lys Leu Leu Ile Tyr Ala Ala Ser Asn Leu Glu Ser
                   50                  55                  60
   Gly Ile Pro Ala Arg Phe Ser Gly Ser Gly Ser Gly Thr Asp Phe
                   65                  70                  75
   Thr Leu Asn Ile His Pro Val Glu Glu Glu Asp Ala Ala Thr Tyr
                   80                  85                  90
   Tyr Cys Gln Gln Ser Asn Glu Asp Pro Phe Thr Phe Gly Ala Gly
                   95                  100                 105
   Thr
   106
(2) INFORMATION FOR SEQ ID NO:7:
   (i) SEQUENCE CHARACTERISTICS:
      (A) LENGTH: 137 amino acids
      (B) TYPE: amino acid
      (D) TOPOLOGY: linear
   (xi) SEQUENCE DESCRIPTION: SEQ ID NO:7:
   Asp Val Gln His Gln Glu Ser Glu Pro Asp Leu Val Lys Pro Ser
    1               5                  10                  15
   Gln Ser Leu Ser Leu Thr Cys Thr Val Thr Gly Tyr Ser Ile Thr
                   20                  25                  30
   Ser Gly Tyr Asn Arg His Trp Ile Arg Gln Phe Pro Gly Asn Lys
                   35                  40                  45
   Leu Glu Trp Met Gly Tyr Ile His Tyr Ser Gly Ser Thr Asn Tyr
                   50                  55                  60
   Asn Pro Ser Leu Lys Arg Arg Ile Ser Ile Thr Arg Asp Thr Ser
                   65                  70                  75
   Lys Asn Gln Phe Phe Leu Gln Leu Asn Ser Val Thr Thr Glu Asp
                   80                  85                  90
   Thr Ala Thr Tyr Tyr Cys Ala Arg Gly Ser Ile Tyr Tyr Tyr Gly
                   95                 100                 105
   Ser Arg Tyr Arg Tyr Phe Asp Val Trp Gly Ala Gly Thr Thr Val
                  110                 115                 120
   Thr Val Ser Ser Ala Lys Arg His Pro His Leu Ser Ile His Trp
                  125                 130                 135
   Pro Gly
       137
(2) INFORMATION FOR SEQ ID NO:8:
   (i) SEQUENCE CHARACTERISTICS:
      (A) LENGTH: 453 amino acids
      (B) TYPE: amino acid
      (D) TOPOLOGY: linear
   (xi) SEQUENCE DESCRIPTION: SEQ ID NO:8:
   Glu Val Gln Leu Val Glu Ser Gly Gly Gly Leu Val Gln Pro Gly
   1 5 10 15
   Gly Ser Leu Arg Leu Ser Cys Ala Val Ser Gly Tyr Ser Ile Thr
   20 25 30
   Ser Gly Tyr Ser Trp Asn Trp Ile Arg Gln Ala Pro Gly Lys Gly
   35 40 45
   Leu Glu Trp Val Ala Ser Ile Thr Tyr Asp Gly Ser Thr Asn Tyr
   50 55 60
   Ala Asp Ser Val Lys Gly Arg Phe Thr Ile Ser Arg Asp Asp Ser
   65 70 75
   Lys Asn Thr Phe Tyr Leu Gln Met Asn Ser Leu Arg Ala Glu Asp
   80 85 90
   Thr Ala Val Tyr Tyr Cys Ala Arg Gly Ser His Tyr Phe Gly His
   95 100 105
   Trp His Phe Ala Val Trp Gly Gln Gly Thr Leu Val Thr Val Ser
   110 115 120
   Ser Ala Ser Thr Lys Gly Lys Gly Pro Ser Val Phe Pro Leu Ala
   125 130 135
   Pro Ser Ser Lys Ser Thr Ser Gly Gly Thr Ala Ala Leu Gly Cys
   140 145 150
   Leu Val Lys Asp Tyr Phe Pro Glu Pro Val Thr Val Ser Trp Asn
   155 160 165
   Ser Gly Ala Leu Thr Ser Gly Val His Thr Phe Pro Ala Val Leu
   170 175 180
   Gln Ser Ser Gly Leu Tyr Ser Leu Ser Ser Val Val Thr Val Pro
   185 190 195
   Ser Ser Ser Leu Gly Thr Gln Thr Tyr Ile Cys Asn Val Asn His
   200 205 210
   Lys Pro Ser Asn Thr Lys Val Asp Lys Lys Val Glu Pro Lys Ser
   215 220 225
   Cys Asp Lys Thr His Thr Cys Pro Pro Cys Pro Ala Pro Glu Leu
   230 235 240
   Leu Gly Gly Pro Ser Val Phe Leu Phe Pro Pro Lys Pro Lys Asp
   245 250 255
   Thr Leu Met Ile Ser Arg Thr Pro Glu Val Thr Cys Val Val Val
   260 265 270
   Asp Val Ser His Glu Asp Pro Glu Val Lys Phe Asn Trp Tyr Val
   275 280 285
   Asp Gly Val Glu Val His Asn Ala Lys Thr Lys Pro Arg Glu Glu
   290 295 300
   Gln Tyr Asn Ser Thr Tyr Arg Val Val Ser Val Leu Thr Val Leu
   305 310 315
   His Gln Asp Trp Leu Asn Gly Lys Glu Tyr Lys Cys Lys Val Ser
   320 325 330
   Asn Lys Ala Leu Pro Ala Pro Ile Glu Lys Thr Ile Ser Lys Ala
   335 340 345
   Lys Gly Gln Pro Arg Glu Pro Gln Val Tyr Thr Leu Pro Pro Ser
   350 355 360
   Arg Glu Glu Met Thr Lys Asn Gln Val Ser Leu Thr Cys Leu Val
   365 370 375
   Lys Gly Phe Tyr Pro Ser Asp Ile Ala Val Glu Trp Glu Ser Asn
   380 385 390
   Gly Gln Pro Glu Asn Asn Tyr Lys Thr Thr Pro Pro Val Leu Asp
   395 400 405
   Ser Asp Gly Ser Phe Phe Leu Tyr Ser Lys Leu Thr Val Asp Lys
   410 415 420
   Ser Arg Trp Gln Gln Gly Asn Val Phe Ser Cys Ser Val Met His
   425 430 435
   Glu Ala Leu His Asn His Tyr Thr Gln Lys Ser Leu Ser Leu Ser
   440 445 450
   Pro Gly Lys
   453
(2) INFORMATION FOR SEQ ID NO:9:
   (i) SEQUENCE CHARACTERISTICS:
      (A) LENGTH: 218 amino acids
      (B) TYPE: amino acid
      (D) TOPOLOGY: linear
   (xi) SEQUENCE DESCRIPTION: SEQ ID NO:9:
   Asp Ile Gln Leu Thr Gln Ser Pro Ser Ser Leu Ser Ala Ser Val
   1 5 10 15
   Gly Asp Arg Val Thr Ile Thr Cys Arg Ala Ser Gln Ser Val Asp
   20 25 30
   Tyr Asp Gly Asp Ser Tyr Met Asn Trp Tyr Gln Gln Lys Pro Gly
   35 40 45
   Lys Ala Pro Lys Leu Leu Ile Tyr Ala Ala Ser Tyr Leu Glu Ser
   50 55 60
   Gly Val Pro Ser Arg Phe Ser Gly Ser Gly Ser Gly Thr Asp Phe
   65 70 75
   Thr Leu Thr Ile Ser Ser Leu Gln Pro Glu Asp Phe Ala Thr Tyr
   80 85 90
   Tyr Cys Gln Gln Ser His Glu Asp Pro Tyr Thr Phe Gly Gln Gly
   95 100 105
   Thr Lys Val Glu Ile Lys Arg Thr Val Ala Ala Pro Ser Val Phe
   110 115 120
   Ile Phe Pro Pro Ser Asp Glu Gln Leu Lys Ser Gly Thr Ala Ser
   125 130 135
   Val Val Cys Leu Leu Asn Asn Phe Tyr Pro Arg Glu Ala Lys Val
   140 145 150
   Gln Trp Lys Val Asp Asn Ala Leu Gln Ser Gly Asn Ser Gln Glu
   155 160 165
   Ser Val Thr Glu Gln Asp Ser Lys Asp Ser Thr Tyr Ser Leu Ser
   170 175 180
   Ser Thr Leu Thr Leu Ser Lys Ala Asp Tyr Glu Lys His Lys Val
   185 190 195
   Tyr Ala Cys Glu Val Thr His Gln Gly Leu Ser Ser Pro Val Thr
   200 205 210
   Lys Ser Phe Asn Arg Gly Glu Cys
   215 218
(2) INFORMATION FOR SEQ ID NO:10:
   (i) SEQUENCE CHARACTERISTICS:
      (A) LENGTH: 8 amino acids
      (B) TYPE: amino acid
      (D) TOPOLOGY: linear
   (xi) SEQUENCE DESCRIPTION: SEQ ID NO:10:
   Phe Asp Leu Phe Ile Arg Lys Ser
   1 5 8
(2) INFORMATION FOR SEQ ID NO:11:
   (i) SEQUENCE CHARACTERISTICS:
      (A) LENGTH: 9 amino acids
      (B) TYPE: amino acid
      (D) TOPOLOGY: linear
   (xi) SEQUENCE DESCRIPTION: SEQ ID NO:11:
   Lys Asp Thr Leu Met Ile Ser Arg Thr
   1 5 9
(2) INFORMATION FOR SEQ ID NO:12:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:12:
   Ala Pro Ser Lys Gly Thr
   1 5 6
(2) INFORMATION FOR SEQ ID NO:13:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:13:
   Ser His Glu Asp Pro Gln
   1 5 6
(2) INFORMATION FOR SEQ ID NO:14:
   (i) SEQUENCE CHARACTERISTICS:
      (A) LENGTH: 11 amino acids
      (B) TYPE: amino acid
      (D) TOPOLOGY: linear
   (xi) SEQUENCE DESCRIPTION: SEQ ID NO:14:
   Ser Arg Ala Ser Gly Lys Pro Val Asn His Ser
   1 5 10 11
(2) INFORMATION FOR SEQ ID NO:15:
   (i) SEQUENCE CHARACTERISTICS:
      (A) LENGTH: 11 amino acids
      (B) TYPE: amino acid
      (D) TOPOLOGY: linear
   (xi) SEQUENCE DESCRIPTION: SEQ ID NO:15:
   Tyr Val Asp Gly Val Gln Val His Asn Ala Lys
   1 5 10 11
(2) INFORMATION FOR SEQ ID NO:16:
   (i) SEQUENCE CHARACTERISTICS:
      (A) LENGTH: 10 amino acids
      (B) TYPE: amino acid
      (D) TOPOLOGY: linear
   (xi) SEQUENCE DESCRIPTION: SEQ ID NO:16:
   Gly Thr Arg Asp Trp Ile Glu Gly Glu Thr
   1 5 10
(2) INFORMATION FOR SEQ ID NO:17:
   (i) SEQUENCE CHARACTERISTICS:
      (A) LENGTH: 10 amino acids
      (B) TYPE: amino acid
      (D) TOPOLOGY: linear
   (xi) SEQUENCE DESCRIPTION: SEQ ID NO:17:
   Leu His Gln Asp Trp Leu Asp Gly Lys Glu
   1 5 10
(2) INFORMATION FOR SEQ ID NO:18:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:18:
   Arg Ala Leu Met
   1 4
(2) INFORMATION FOR SEQ ID NO:19:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:19:
   Ala Pro Ile Glu
    1           4
(2) INFORMATION FOR SEQ ID NO:20:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:20:
   Lys Glu Glu Lys Gln Arg
    1               5   6
(2) INFORMATION FOR SEQ ID NO:21:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:21:
   Pro Arg Glu Gln Gln Tyr
    1               5   6
(2) INFORMATION FOR SEQ ID NO:22:
   (i) SEQUENCE CHARACTERISTICS:
      (A) LENGTH: 5 amino acids
      (B) TYPE: amino acid
      (D) TOPOLOGY: linear
   (xi) SEQUENCE DESCRIPTION: SEQ ID NO:22:
   Gln Cys Arg Val Thr
    1               5
(2) INFORMATION FOR SEQ ID NO:23:
   (i) SEQUENCE CHARACTERISTICS:
      (A) LENGTH: 5 amino acids
      (B) TYPE: amino acid
      (D) TOPOLOGY: linear
   (xi) SEQUENCE DESCRIPTION: SEQ ID NO:23:
   Ala Cys Ala Val Ala
   1 5
(2) INFORMATION FOR SEQ ID NO:24:
   (i) SEQUENCE CHARACTERISTICS:
      (A) LENGTH: 8 amino acids
      (B) TYPE: amino acid
      (D) TOPOLOGY: linear
   (xi) SEQUENCE DESCRIPTION: SEQ ID NO:24:
   Gln Lys His Trp Leu Ser Asp Arg
   1 5 8
(2) INFORMATION FOR SEQ ID NO:25:
   (i) SEQUENCE CHARACTERISTICS:
      (A) LENGTH: 8 amino acids
      (B) TYPE: amino acid
      (D) TOPOLOGY: linear
   (xi) SEQUENCE DESCRIPTION: SEQ ID NO:25:
   Ala Ala Ala Trp Leu Ala Ala Ala
   1 5 8
(2) INFORMATION FOR SEQ ID NO:26:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:26:
   Tyr Asp Gly Asp
   1 4
(2) INFORMATION FOR SEQ ID NO:27:
   (i) SEQUENCE CHARACTERISTICS:
      (A) LENGTH: 8 amino acids
      (B) TYPE: amino acid
      (D) TOPOLOGY: linear
   (xi) SEQUENCE DESCRIPTION: SEQ ID NO:27:
   Phe Asp Leu Phe Ile Arg Lys Ser
    1               5          8
(2) INFORMATION FOR SEQ ID NO:28:
   (i) SEQUENCE CHARACTERISTICS:
      (A) LENGTH: 9 amino acids
      (B) TYPE: amino acid
      (D) TOPOLOGY: linear
   (xi) SEQUENCE DESCRIPTION: SEQ ID NO:28:
   Lys Asp Thr Leu Met Ile Ser Arg Thr
    1               5               9
(2) INFORMATION FOR SEQ ID NO:29:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:29:
   Phe Asp Leu Phe
    1           4
(2) INFORMATION FOR SEQ ID NO:30:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:30:
   Gln Asp Leu His
    1           4
(2) INFORMATION FOR SEQ ID NO:31:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:31:
   Ala Pro Ser Lys Gly Thr
    1               5   6
(2) INFORMATION FOR SEQ ID NO:32:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:32:
   Ser His Glu Asp Pro Gln
    1               5   6
(2) INFORMATION FOR SEQ ID NO:33:
   (i) SEQUENCE CHARACTERISTICS:
      (A) LENGTH: 11 amino acids
      (B) TYPE: amino acid
      (D) TOPOLOGY: linear
   (xi) SEQUENCE DESCRIPTION: SEQ ID NO:33:
   Ser Arg Ala Ser Gly Lys Pro Val Asn His Ser
    1               5                  10  11
(2) INFORMATION FOR SEQ ID NO:34:
   (i) SEQUENCE CHARACTERISTICS:
      (A) LENGTH: 11 amino acids
      (B) TYPE: amino acid
      (D) TOPOLOGY: linear
   (xi) SEQUENCE DESCRIPTION: SEQ ID NO:34:
   Tyr Val Asp Gly Val Gln Val His Asn Ala Lys
   1 5 10 11
(2) INFORMATION FOR SEQ ID NO:35:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:35:
   Ser Arg Ala Ser Gly Lys
   1 5 6
(2) INFORMATION FOR SEQ ID NO:36:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:36:
   Ala Ala Ala Ala Gly Ala
   1 5 6
(2) INFORMATION FOR SEQ ID NO:37:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:37:
   Lys Glu Glu Lys Gln Arg
   1 5 6
(2) INFORMATION FOR SEQ ID NO:38:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:38:
   Pro Arg Glu Gln Gln Tyr
   1 5 6
(2) INFORMATION FOR SEQ ID NO:39:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:39:
   Lys Glu Glu Lys Gln Arg
   1 5 6
(2) INFORMATION FOR SEQ ID NO:40:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:40:
   Ala Glu Ala Lys Ala Arg
   1 5 6
(2) INFORMATION FOR SEQ ID NO:41:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:41:
   Lys Glu Glu Lys Gln Arg
   1 5 6
(2) INFORMATION FOR SEQ ID NO:42:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:42:
   Lys Ala Glu Ala Gln Ala
    1               5   6
(2) INFORMATION FOR SEQ ID NO:43:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:43:
   Lys Glu Glu Lys Gln Arg
    1               5   6
(2) INFORMATION FOR SEQ ID NO:44:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:44:
   Ala Ala Glu Ala Gln Ala
    1               5   6
(2) INFORMATION FOR SEQ ID NO:45:
   (i) SEQUENCE CHARACTERISTICS:
      (A) LENGTH: 10 amino acids
      (B) TYPE: amino acid
      (D) TOPOLOGY: linear
   (xi) SEQUENCE DESCRIPTION: SEQ ID NO:45:
   Gly Thr Arg Asp Trp Ile Glu Gly Glu Thr
    1               5                   10
(2) INFORMATION FOR SEQ ID NO:46:
   (i) SEQUENCE CHARACTERISTICS:
      (A) LENGTH: 10 amino acids
      (B) TYPE: amino acid
      (D) TOPOLOGY: linear
   (xi) SEQUENCE DESCRIPTION: SEQ ID NO:46:
   Leu His Gln Asp Trp Leu Asp Gly Lys Glu
   1 5 10
(2) INFORMATION FOR SEQ ID NO:47:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:47:
   Glu Gly Glu Thr
   1 4
(2) INFORMATION FOR SEQ ID NO:48:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:48:
   Ala Gly Ala Ala
   1 4
(2) INFORMATION FOR SEQ ID NO:49:
   (i) SEQUENCE CHARACTERISTICS:
      (A) LENGTH: 9 amino acids
      (B) TYPE: amino acid
      (D) TOPOLOGY: linear
   (xi) SEQUENCE DESCRIPTION: SEQ ID NO:49:
   Thr Arg Asp Trp Ile Glu Gly Glu Thr
   1 5 9
(2) INFORMATION FOR SEQ ID NO:50:
   (i) SEQUENCE CHARACTERISTICS:
      (A) LENGTH: 9 amino acids
      (B) TYPE: amino acid
      (D) TOPOLOGY: linear
   (xi) SEQUENCE DESCRIPTION: SEQ ID NO:50:
   His Gln Asp Trp Leu Asp Gly Lys Glu
   1 5 9
(2) INFORMATION FOR SEQ ID NO:51:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:51:
   Glu Gly Glu Thr
   1 4
(2) INFORMATION FOR SEQ ID NO:52:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:52:
   Asp Gly Lys Glu
   1 4
(2) INFORMATION FOR SEQ ID NO:53:
   (i) SEQUENCE CHARACTERISTICS:
      (A) LENGTH: 5 amino acids
      (B) TYPE: amino acid
      (D) TOPOLOGY: linear
   (xi) SEQUENCE DESCRIPTION: SEQ ID NO:53:
   Gln Cys Arg Val Thr
   1 5
(2) INFORMATION FOR SEQ ID NO:54:
   (i) SEQUENCE CHARACTERISTICS:
      (A) LENGTH: 5 amino acids
      (B) TYPE: amino acid
      (D) TOPOLOGY: linear
   (xi) SEQUENCE DESCRIPTION: SEQ ID NO:54:
   Ala Cys Ala Val Ala
    1               5
(2) INFORMATION FOR SEQ ID NO:55:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:55:
   Arg Ala Leu Met
    1           4
(2) INFORMATION FOR SEQ ID NO:56:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:56:
   Ala Pro Ile Glu
    1           4
(2) INFORMATION FOR SEQ ID NO:57:
   (i) SEQUENCE CHARACTERISTICS:
      (A) LENGTH: 8 amino acids
      (B) TYPE: amino acid
      (D) TOPOLOGY: linear
   (xi) SEQUENCE DESCRIPTION: SEQ ID NO:57:
   Gln Lys His Trp Leu Ser Asp Arg
    1               5           8
(2) INFORMATION FOR SEQ ID NO:58:
   (i) SEQUENCE CHARACTERISTICS:
      (A) LENGTH: 8 amino acids
      (B) TYPE: amino acid
      (D) TOPOLOGY: linear
   (xi) SEQUENCE DESCRIPTION: SEQ ID NO:58:
   Ala Ala Ala Trp Leu Ala Ala Ala
    1               5           8
(2) INFORMATION FOR SEQ ID NO:59:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:59:
   Pro Arg Ala Ala
    1           4
(2) INFORMATION FOR SEQ ID NO:60:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:60:
   Gln Pro Arg Glu
    1           4
(2) INFORMATION FOR SEQ ID NO:61:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:61:
   Ala Ser Pro Ser Gln Thr
   1 5 6
(2) INFORMATION FOR SEQ ID NO:62:
   (i) SEQUENCE CHARACTERISTICS:
      (A) LENGTH: 5 amino acids
      (B) TYPE: amino acid
      (D) TOPOLOGY: linear
   (xi) SEQUENCE DESCRIPTION: SEQ ID NO:62:
   Leu His Asn His Tyr
   1 5
(2) INFORMATION FOR SEQ ID NO:63:
   (i) SEQUENCE CHARACTERISTICS:
      (A) LENGTH: 5 amino acids
      (B) TYPE: amino acid
      (D) TOPOLOGY: linear
   (xi) SEQUENCE DESCRIPTION: SEQ ID NO:63:
   Ser Pro Ser Gln Thr
   1 5
(2) INFORMATION FOR SEQ ID NO:64:
   (i) SEQUENCE CHARACTERISTICS:
      (A) LENGTH: 5 amino acids
      (B) TYPE: amino acid
      (D) TOPOLOGY: linear
   (xi) SEQUENCE DESCRIPTION: SEQ ID NO:64:
   Ala Pro Ala Ala Ala
   1 5

## Description

### Background of the Invention

This invention relates to amino acid sequence variant anti-IgE antibodies,
und especially to humanised antibodies capable of differential binding to FcεRI and FcεRII.

IgE is a member of the immunoglobulin family that mediates allergic responses such as asthma, food allergies, type 1 hypersensitivity and the familiar sinus inflammation suffered on a widespread basis. IgE is secreted by, and expressed on the surface of, B-cells. IgE synthesized by B-cells is anchored in the B-cell membrane by a transmembrane domain linked to the mature IgE sequence by a short membrane binding region. IgE also is bound to B-cells (and monocytes, eosinophils and platelets) through its Fc region to a low affinity IgE receptor (FcεRII, hereafter "FCEL"). Upon exposure of a mammal to an allergen, B-cells are clonally amplified which synthesize IgE that binds the allergen. This IgE in turn is released into the circulation by the B-cells where it is bound by B-cells (through the FCEL) and by mast cells and basophils through the so-called high affinity receptor (FcεRi, hereinafter "FCEH") found on the surface of the mast cells and basophils. Such mast cells and basophils are thereby sensitized for allergen. The next exposure to the allergen cross-links the FcεRI on these cells and thus activates their release of histamine and other factors which are responsible for clinical hypersensitivity and anaphylaxis.

The art has reported antibodies capable of binding to PCEL-bound IgE but not IgE located on FCEH (see for example WO 89/00138 and US patent 4,940,782). These antibodies are disclosed to be clinically advantageous because they bind to IgE found on B-cells or circulating free in the body, but do not bind to FCEH and thus will not activate mast cells or basophils. In addition, various amino acid sequence variants of immunoglobulins are known, e.g., "chimeric" and "humanized" antibodies (see, for example, U.S. Patent 4,816,567; WO 91/09968; EP 452,508; and WO 91/16927). Humanized antibodies are immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues which are found neither in the recipient antibody nor in the imported CDR or framework sequences. These modifications are made to further refine and optimize antibody performance as will be more further described *infra.* Also known *per se* are monovalent and bispecific antibodies.

It is generally understood that FCEH, like FCEL, binds to recognition site(s) in the IgE constant (Fc) domain. The IgE recognition site(s) for the two receptors are poorly defined, despite considerable effort in the past directed to the problem.

Over the past decade several studies have been undertaken to determine which portion of the IgE molecule is involved in binding to FcεRI and FcεRII. Essentially three approaches have been tried. First, peptides corresponding to specific portions of IgE sequence have been used as either competitive inhibitors of IgE-receptor binding (Burt *et al.,* Eur. J. Immun, **17**:437-440 [1987]; Helm *et al.*, Nature, **331:**180-183 [1988]; Helm *et al.,* Proc. Natl. Acad. Sci., **86:**9465-9469 [1989]; Vercelli *et al.,* Nature, **338:**649-651 [1989]; Nio *et al.*, Peptide Chemistry, 203-208 [1990]) or to elicit anti-IgE antibodies which would block IgE-receptor interaction (Burt *et al*., Molec. Immun. **24**:379-389 [1987]; Robertson *et al.,* Molec. Immun., **25**:103-113 [1988]; Baniyash *et al.,* Molec. Immun. **25:**705-711 [1988]). The most effective competitive peptide was a sequence that was 1000-fold less active than IgE (Burt *et al.,* Eur. J. Immun., **17**:437-440 [1987]).

Helm *et al.,* Proc. Natl. Acad. Sci., **86**:9465-9469 (1989) found that a peptide corresponding to IgE residues 329-409 blocked *in vivo* sensitization of human basophil granulocytes with human IgE antibodies. Further studies indicated that residues 395-409 were not essential for binding of the 329-409 peptide to FcεRI (Helm *et al.,* Proc. Natl. Acad Sci., **86**:9465-9469 [1989]). Note that the IgE sequence variants described below had the sequence of Padlan *et al.,* Mol. Immun., **23**:1063 (1986), but that the immunoglobulin residue numbers used herein are those of Kabat *et al.*, Sequences of Proteins of Immunological Interest (National Institutes of Health, Bethesda, Md. 1987).

Vercelli *et al.,* Nature, **338**:649-651 (1989) used recombinant IgE peptides as well as anti-Fcε monoclonal antibodies to investigate the B-cell (FcεRII) binding site of human IgE. They concluded that the FcεRII binding site is in Fcε3 near K399-V402.

Burt *et al.,* Eur. J. Immun., **17:**437-440 (1987) investigated seven peptides for competition against rat IgE in binding to rat mast cells. Their most active peptide, p129, was 1000-fold less active than IgE. p129 corresponds to human sequence 439-453 which includes loop EF. Another of their peptides, p130, corresponding to residues 396-419 in the Fcε3 domain, had no activity.

Robertson *et al.,* Molec. Immun., **25:**103-113 (1988) assessed IgE binding by sequence-directed antibodies induced by several synthetic peptides. They concluded that the sequence defined by their ε-peptide-4 (corresponding to residues 446-460), was not significantly involved in receptor binding while the sequence defined by their ε-peptide-3 (corresponding to residues 387-401), was likely to be proximal to the IgE-receptor recognition site.

Nio *et al.,* Peptide Chemistry, 203-208 (1990) evaluated numerous peptides with respect to their ability to inhibit histamine release by human basophils *in vitro.* Only one peptide (peptide 2, Table 1), exhibited specific inhibition; this peptide encompassed residues 376-388. However, a larger peptide which incorporated this sequence (peptide 3, Table 1), had no inhibitory activity.

Second, mutations in IgE have been partially explored. Schwarzbaum et al., Eur. J. Immun., 19:1015-1023 [1989] (*supra*) found that a point mutant P404H (P442H by the numbering system used herein) had 2-fold reduced affinity for FcεRI on rat basophilic leukemia (RBL) cells, but the interpretation of this finding is controversial (Weetall *et al.,* J. Immunol., **145**:3849-3854 [1990]).

Third, chimeric molecules have been constructed. Human IgE does not bind to the murine receptor (Kulczycki Jr., *et al.,* J. Exp. Med., **139:**600-616 [1974]) while rodent IgE binds to the human receptor with a reduced affinity (Conrad, *et al.*, J. Immun., **130**:327-333 [1983]); human IgG1 does not bind to IgE receptors (Weetall *et al.,* J. Immun., **145**:3849-3854 [1990]). Based on these observations, several groups have constructed human-murine chimeras or human IgE-IgG chimeras. Weetall *et al.,* J. Immun., **145**:3849-3854 (1990) made a series of human IgG1-murine IgE chimeras and concluded that the Fcε2 and Fcε3 domains are involved in binding murine FcεRI while the Fcε4 domain is unlikely to be involved in binding to murine FcεRI (but may possibly be involved in binding to FcεRII). However, these conclusions are uncertain since they rest primarily on *lack* of binding by chimeras and three of five chimeras lacked some interchain disulfide bonds.

Nissim *et al.,* EMBO J., **10**:101-107 (1991) constructed a series of human-murine IgE chimeras and measured binding to RBL cells and concluded that the portion of IgE which binds with high affinity to the specialized Fcε receptor on RBL cells could be assigned to Fcε3.

The results reported by these authors (e.g. Helm *et al.* and Burt *et al*.) are inconsistent. Further, in the case of anti-IgE antibodies it is difficult to eliminate the possibility of nonspecific blocking due to steric hindrance (Schwarzbaum *et al.,* Eur. J. Immun., **19**:1015-1023 [1989]). It is apparent that considerable confusion exists in the art as to the domains of IgE Fc which are involved in the binding of IgE to FCEH or in the maintenance of IgE conformation responsible for IgE binding to FCEH.

### Summary of the invention

Broadly, the present invention related to antibodies which are capable of binding to FcεRII bound IgE but which are substantially incapable of binding to FCεRI-bound IgE.

According to one aspect, the present invention provides an antibody which is capable of binding to FcεRII-bound IgE but is substantially incapable of binding to FcεRI-bound IgE, comprising a human recipient antibody into which have been substituted at one or more of positions 30, 30b, 30d, 33, 53, 91, 92, 93 and 94 in the light chain and positions 27, 28, 29, 29a, 31, 33, 34, 50, 52, 53, 54, 55, 58, 95, 97, 98, 99, 100 and 101 in the heavy chain, residues from analogous positions in donor antibody MAE11, MAE13 or MAE15 having the light and heavy chain amino acid sequences as set out in SEQ ID NO:2 to 7, or a donor antibody having the characteristics possessed by the MAE11 antibody, in particular in binding soluble IgE, binding IgE-bearing B cells, blocking IgE binding to FcεRI and FcεRII, inhibiting in vitro IgE production and failing to bind to IgE coated basophils.

One preferred embodiment is an antibody comprising the heavy and light chain sequences of humae11ver.1, 2, 3, 4, 5, 6, 7, 7a, 8, 8a, 8b or 9, wherein humae11ver.1 has the heavy and light chain amino acid sequences as set out in SEQ ID NO: 8 and 9, and humae11ver.2-9 have the heavy and light chain amino acid sequences of humae11ver.1, further incorporating the modifications shown in Table 5.

Another preferred embodiment is an antibody comprising heavy and light chain amino acid sequences of humae11ver.1 as set out in SEQ ID NO: 8 and 9, said heavy chain sequence being substituted at position 60 with asparagine, at position 61 with proline and position 67 with isoleucine.

The differential binding polypeptides of this invention are useful in diagnostic procedures for IgE receptors or in the therapy of IgE-mediated disorders such as allergies. They also are useful in preparing antibodies capable of binding regions of IgE that participate in receptor binding.

In an embodiment of this invention, variant anti-IgE antibodies are provided for use in diagnosis or for the therapy or prophylaxis of allergic and other IgE-mediated disorders. In particular embodiments of this invention anti-IgE variant antibodies are provided in which one or more human (recipient) light chain residues 4, 13, 19, 24, 29, 30, 33, 55, 57, 58, 78, 93, 94, or 104, or heavy chain residues 24, 37, 48, 49, 54, 57, 60, 61, 63, 65, 67, 69, 78, 82, 97 or 100 have been modified, preferably by substitution with the residue found in the corresponding position in the donor (generally murine) antibody. In preferred embodiments, the selected residues are light chain 13, 19, 58, 78, or 104, or heavy chain residues 48, 49, 60, 61, 63, 67, 69, 82 or 82c, and most preferably are heavy chain residues 60, 61 or light chain residue 78.

In other embodiments we provide antibodies which are capable of binding FCEL-bound IgE but which are substantially incapable of binding FCEH-bound IgE or inducing histamine release from mast cells or basophils, comprising a human Kabat CDR domain into which has been substituted a positionally analogous residue from a Kabat CDR domain of the murine anti-huIgE antibodies MAE11, MAE13, MAE15 or MAE17. Also provided herein are bispecific antibodies and IgE-monovalent antibodies; and humanized antibodies exhibiting an affinity for IgE which ranges from about 0.1 to 100 times that of MAE11.

### Brief Description of the Figure

FIG. 1 depicts the sequence of human IgE Fcε2 and Fcε3 (SEQ. ID. 1). This particular sequence is from Padlan *et al.*, Molec. Immun., **23**:1063-1075 (1986). Residues are numbered according to Kabat (*supra*). "X" residues are included to align the Padlan IgE sequence with the Kabat numbering scheme. Sequences which were altered in preparing various IgE mutants are underlined; bold numbers below the lines denote the mutant number. β-strand residues are overlined; loop residues are defined by all residues intervening between two β-strands.

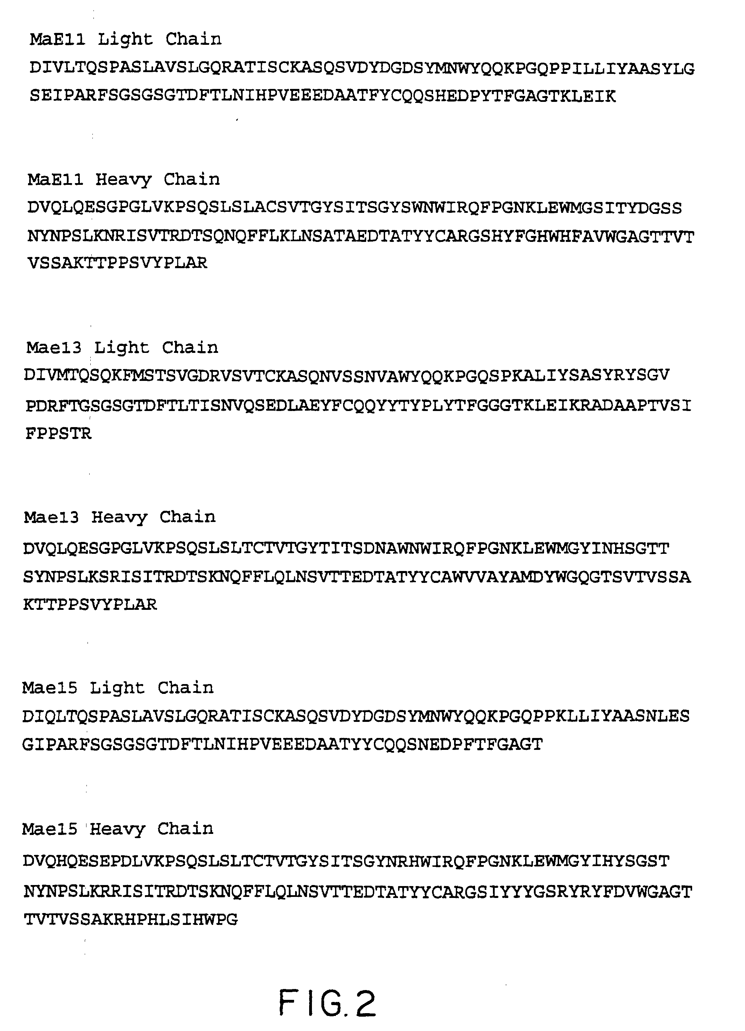

Fig. 2 depicts light and heavy chain sequences for MAE11 (SEQ.ID. 2 and 3), MAE13 (SEQ.ID. 4 and 5) and MAE15 (SEQ.ID. 6 and 7).
Fig. 3 depicts heavy and light chain sequences for HuMae11V1 (SEQ.ID 8 and 9).
Figs. 4a and 4b depicts the percent inhibition of IgE binding to FCEL and FCEH receptors, respectively, by murine monoclonal antibody Mae11 as well as 3 humanized variants (v1, v8 and v9).

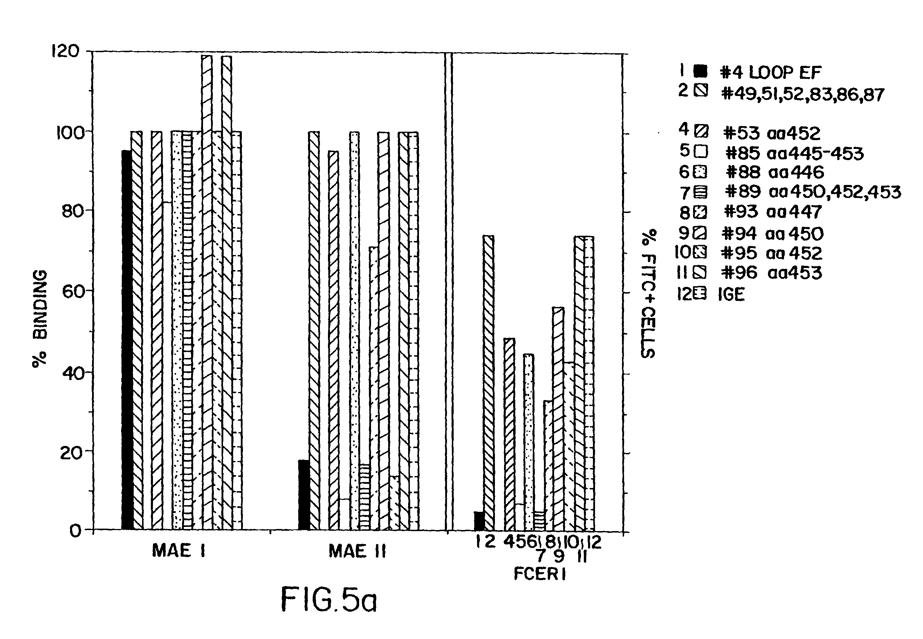

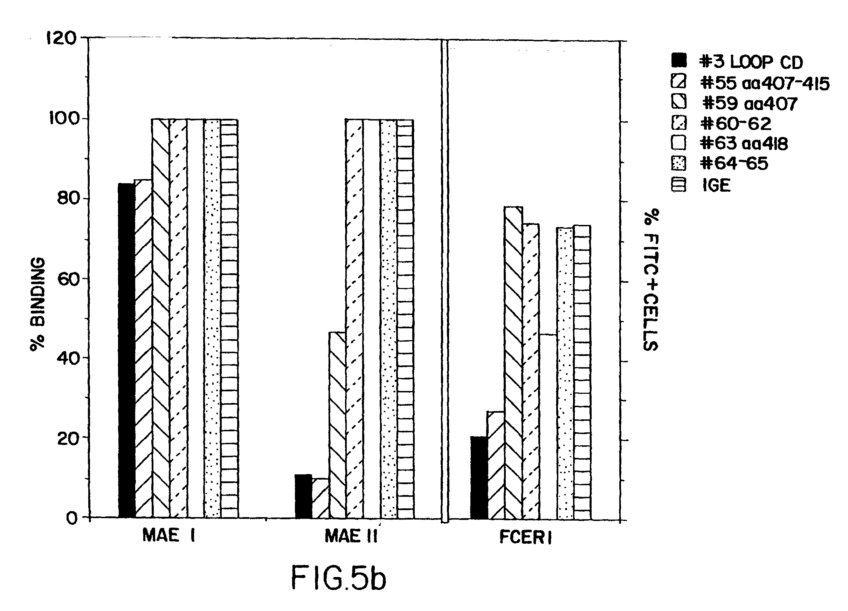

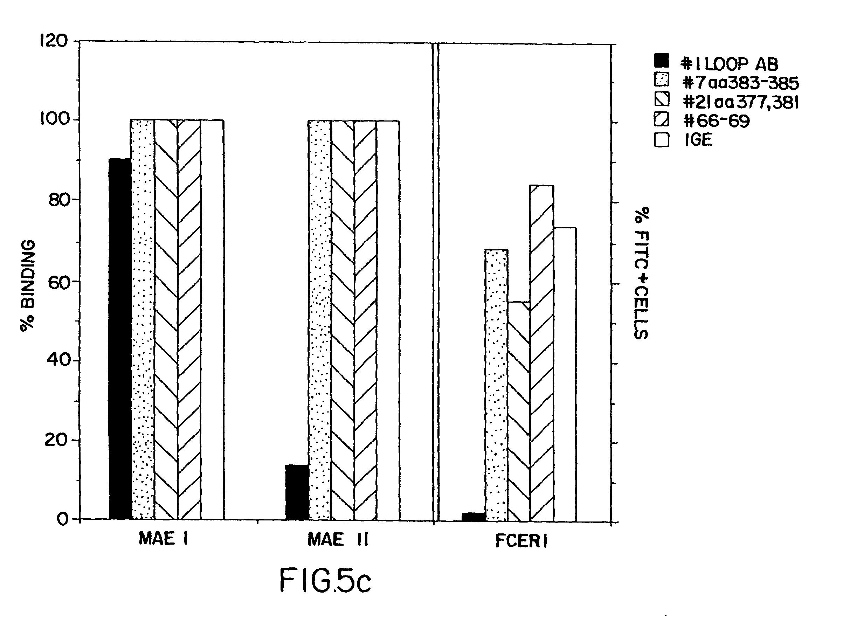

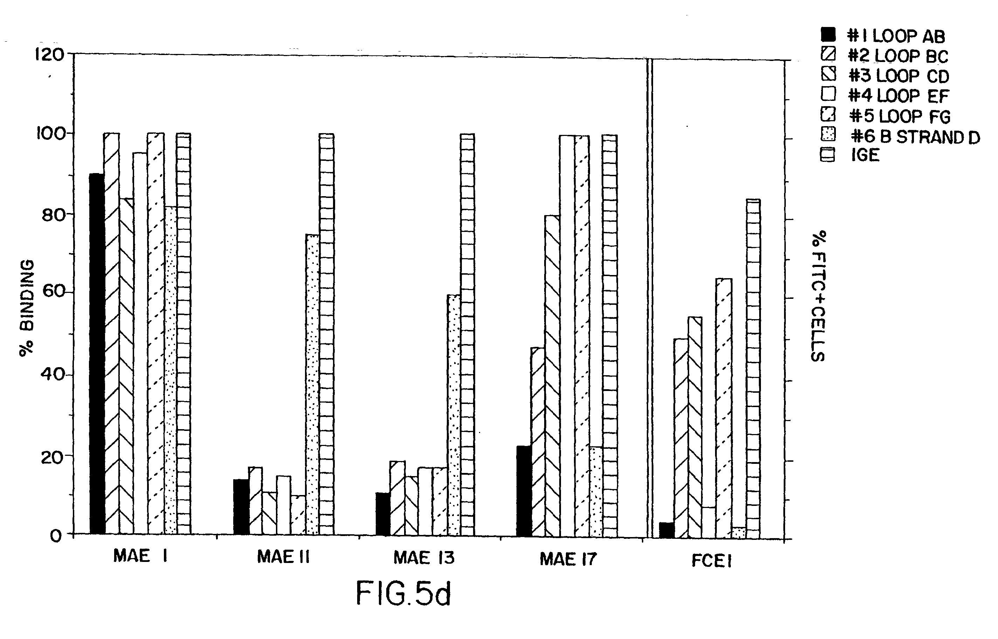

Figs. 5a-5d compare the binding of the MAE11, MAE15 and MAE17 antibodies to various huIgE variants. MAE1 is provided as a control which binds to both B cells and mast cell-bound IgE. The mutants scheduled in the boxes in each figure are identified in Table 11.

### Detailed Description of the Invention

The IgE analogue polypeptides of this invention contain an amino acid sequence which is homologous to that of a naturally occurring IgE and have the ability to bind specifically or differentially to FCEL or FCEH but, in varying degree, not to both. The degree of homology of such polypeptides to wild-type IgE is not critical since only enough IgE sequence needs to be retained to enable the IgE to bind differentially or specifically to one of the two receptors. In general, the polypeptides of this invention will be IgE Fc analogues and will be about from 80% to 99% homologous with a polypeptide sequence of a naturally occurring IgE heavy chain Fc region. Homology is determined by conventional methods in which all substitutions are considered to be nonhomologous (whether conservative or nonconservative) and in which the sequences are aligned to achieve maximal homology.

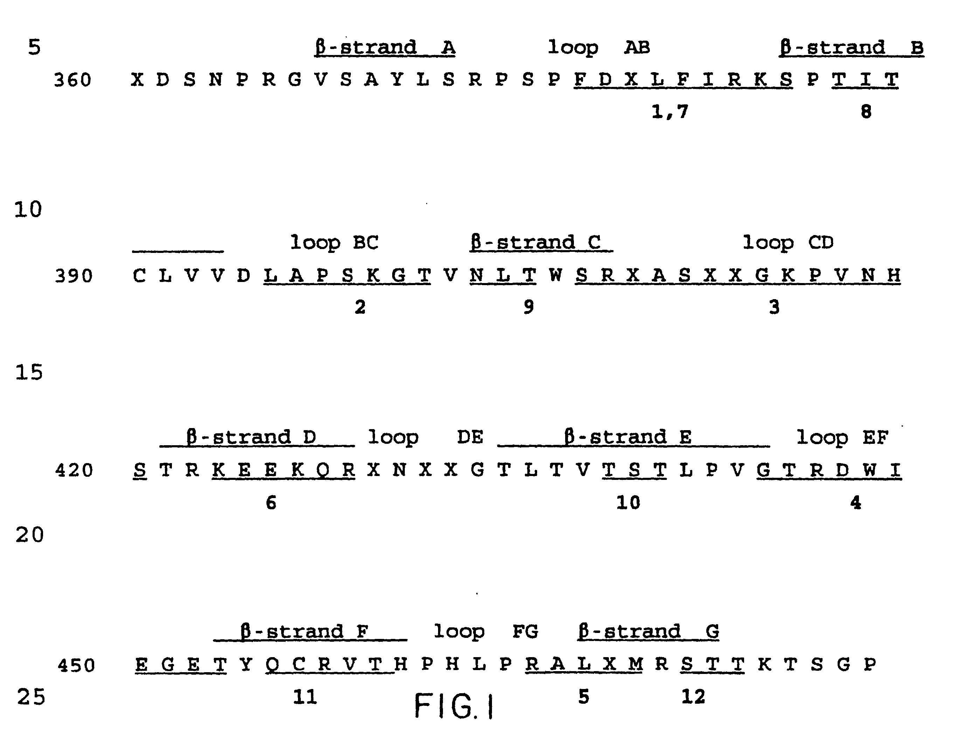

It will be understood that the IgE Fc residue numbers referred to herein are those of Kabat. In applying the residue teachings of this invention to other IgE Fc domains it will be necessary to compare the entire candidate sequence with the Fig. 1 sequence in order to align the residues and correlate the residue numbers. In addition, the identity of certain individual residues at any given Kabat site number may vary from IgE to IgE due to interspecies or allelic divergence. When for example it is stated that substitutions are introduced at residue R383 (human IgE) it will be understood that this means introducing a substitution at the same site in IgE even though this same site (in loop AB) may be located at a different residue number or may be represented in the parental or starting IgE by a residue which is different than that described by Kabat. However, for the sake of clarity and simplicity the residue numbers and identities of the Kabat human IgE heavy chain sequences will be used herein. Note that some Kabat residues were deleted in the Padlan sequence, in which case the Kabat numbering system is preserved by insertion of a spacer residue designated "X" (See Fig. 1 ).

Similarly, the Kabat system is used to designate immunoglobulin residues used in the preparation of variant, e.g. humanized, anti-IgE immunoglobulins such as IgG, IgE, IgA or IgD. In preferred embodiments the recipient human immunoglobulin site is numbered in accord with Kabat subgroups III (V_{H}) consensus and κ subgroup I (V_{L}) consensus sequences. In order to determine which donor residues correspond to these Kabat consensus residues the sequences are maximally aligned, introducing gaps as necessary, using the variable domain cysteine residues as principal guideposts. Note that CDRs vary considerably from antibody to antibody (and by definition will not exhibit homology with the Kabat consensus sequences). Maximal alignment of framework residues (particularly the cysteines) frequently will require the insertion of "spacer" residues in the numbering system, to be used for the Fᵥ region of the donor antibody. For example, the residue "29a" referred to *infra.* This represents an extra residue found in the murine donor antibody V_{H1} CDR for which a counterpart does not exist in the consensus sequence but whose insertion is needed to obtain maximal alignment of consensus and donor sequences. In practice, then, when a humanized antibody (ver. 1) is prepared from this donor it will contain V_{H1} with residue 29a.

The differential binding polypeptides of this invention typically contain about from 5 to 250 residues which are homologous to an IgE heavy chain Fc region, but ordinarily will contain about from 10 to 100 such residues. Usually, the IgE Fc3 and Fc4 regions will be present, with the Fc3 domain providing residues directly involved in receptor binding with Fc4 being present to ensure conformational integrity.

Generally, the IgE is human IgE, although animal IgE such as rat, murine, equine, bovine, feline or porcine IgE is included. As noted above, there will be variation in the residue identities and numbers for these IgEs compared to the Fig. 1 sequence.

FCEH and FCEL are respectively defined to be the high affinity IgE receptor (FCεRI, Ishizaka *et al.,* Immunochemistry, **7**:687-702 [1973]) found on mast cells or basophils, and the low affinity receptor (FCεRII, or CD23) found on cells involved in inflammation such as monocytes, eosinophils and platelets, as well as B-cells (Capron *et al.*, Immun. Today, **7**:15-18 [1986]). FCEH and FCEL include alleles and predetermined amino acid sequence variants thereof which bind IgE. While FCEH contains several polypeptide chains, the binding of candidate polypeptides to its alpha chain is all that needs to be assayed since the alpha chain is the portion of FCEH which binds IgE.

Differential binding means that the polypeptide will bind to one of FCEL or FCEH to the extent of at least about 75% of the degree with which the homologous native IgE binds to that receptor, but will not bind to the other receptor at more than about 20% of the degree that the homologous IgE binds to the other receptor. Binding is determined by the assays of Example 3. Included within this invention are polypeptides that are capable of binding to one of the two receptors to a greater degree than native IgE.

### Variant Anti-huIgE antibodies

Variant anti-huIgE antibodies were produced by first obtaining a group of murine monoclonal antibodies which were capable of binding to FCEL but not to FCEH. 8 such murine monoclonal antibodies, designated MAE10, MAE11, MAE12, MAE13, MAE14, MAE15, MAE16 and MAE17, were obtained by conventional methods involving immunizing mice with human IgE or a polypeptide consisting of residues 315-547 of huIgE and screening for anti-IgE activity.

MAE11/15 and MAE13 recognize different epitopes. It appears that the MAE13 epitope is located three-dimensionally adjacent to a key component of the FCEH binding site of IgE (but does not directly occupy that site) since a slight amount of histamine release will occur at high concentrations of MAE 13 suggesting that some limited antibody mediated crosslinking of FCEH occurs with MAE 13. MAE17 was most effective in suppressing B-cell IgE synthesis despite the fact that MAE11 and MAE13 exhibited greater IgE affinity. This may be attributed to its ability to mediate complement fixation (it possessed an IgG2a isotope, thus containing an Fc capable of eliciting effector function).

MAE11 and MAE15 are believed to recognize the same IgE epitope. Each antibody shared certain unusual features in its amino acid sequence. For example, CDR1 of the light chain of each contained 3 aspartic acid residues. CDR3 of the heavy chains of MAE11 and MAE15 contained 3 histidine residues (and contained two arginine residues, respectively).

Antibodies such as the foregoing having desired IgE binding characteristics may be further modified. Such modifications fall into two general classes. In the first class the antibodies are modified so that they are monovalent for IgE. This means that only one "arm" of the antibody, i.e., one light-heavy chain fork of the antibody, shall be capable of binding IgE. The remaining Fv "arm" of the antibody (or arms in the case of IgM) is specific for a second (non-IgE) antigen, is not capable of binding any antigen, or is deleted entirely. Thus, the term IgE monovalent covers polyvalent antibodies that are monovalent for IgE. The best results may be obtained with the second alternative, since this would preserve the structure of the antibody most faithfully and would likely confer the longest circulating half-life on the antibody. IgE-monovalent antibodies specific for FCEL bound IgE optimally will comprise sufficient fc domains of the heavy chains to be capable of complement binding and Ig effector functions.

The second antigen recognized by one embodiment of IgE monovalent antibody is one which, when indirectly crosslinked to FCEL by the antibody herein, will not produce any toxic or deleterious response, i.e. the second antigen is not FCEH, and generally is one which is not found in the animal to be treated (in order to avoid undesired absorption of the antibody onto tissues or proteins within the body). Thus, the second antigen ordinarily will not (but may be) FCEL. However, in some circumstances the second antigen will be a protein present in the patient to be treated, e.g. where such proteins are to serve as carriers or depot releases for the therapeutic antibodies herein.

Such IgE-monovalent antibodies are made by methods known per se. For example, DNA encoding the anti-IgE Fv heavy and light chains is ligated to DNA encoding the Fc of a human recipient antibody. In addition, DNA is provided that encodes heavy and light chains for an antibody capable of binding second antigen or an unidentified antigen, or that encodes heavy and light chain having sufficient residues deleted from the CDRs that non-IgE antigen binding no longer can occur. A conventional recombinant host is transformed with all four DNAs and the products recovered. Assuming random chain assortment, a subpopulation of antibody products will contain one arm with anti-IgE heavy and light chain and at least another arm having specificity for second antigen or no antigen. The desired subpopulation then is purified by conventional methods, e.g., immunoaffinity absorption or by molecular sieving. These antibodies also can be made by reduction of the starting antibodies followed by oxidative chain recombination, as has heretofore been employed in the preparation of monovalent antibodies (see for example Glennie *et al.,* Nature **295:**712 (1982]).

In addition to IgE-monovalency, in other embodiments the antibodies are modified so that they contain a maximum proportion of human sequence (commensurate with retention of required or desired activity), i.e., they are converted to chimeras or are humanized. In both instances the functional effect is to place the anti-IgE binding capability of the murine or other donor antibody into a human background to make it as non-immunogenic as possible. General methods are known for making chimeras and for humanizing antibodies (as noted above). A minimal amount of non-human antibody sequence is substituted into the recipient human antibody. Typically, the non-human residues are substituted into the V_{H}, V_{L}, V_{H}-V_{L} interface or framework of the recipient human antibody. Generally, the Kabat CDR's of the humanized antibodies are about 80% and more typically about 90% homologous with the non-human donor CDR's. The V_{H}-V_{L} interface and framework residues of the humanized antibody, on the other hand, are about 80%, ordinarily 90% and preferably about 95% homologous with the recipient human antibody. Homology is determined by maximal alignment of identical residues. The resulting antibody is (a) less immunogenic in humans than a murine antibody and (b) capable of binding to FCEL-bound huIgE but substantially incapable of binding to FCEH-bound huIgE. Such antibodies typically comprise a human antibody which is substituted by an amino acid residue from a complementarity determining region (CDR), VL-VH interface or a framework region of a non-human anti-IgE antibody which is capable of binding. One or more, and preferably all, of the nonhuman CDR's L1, L2, L3, H1, H2 or H3 are substituted into the human antibody recipient.

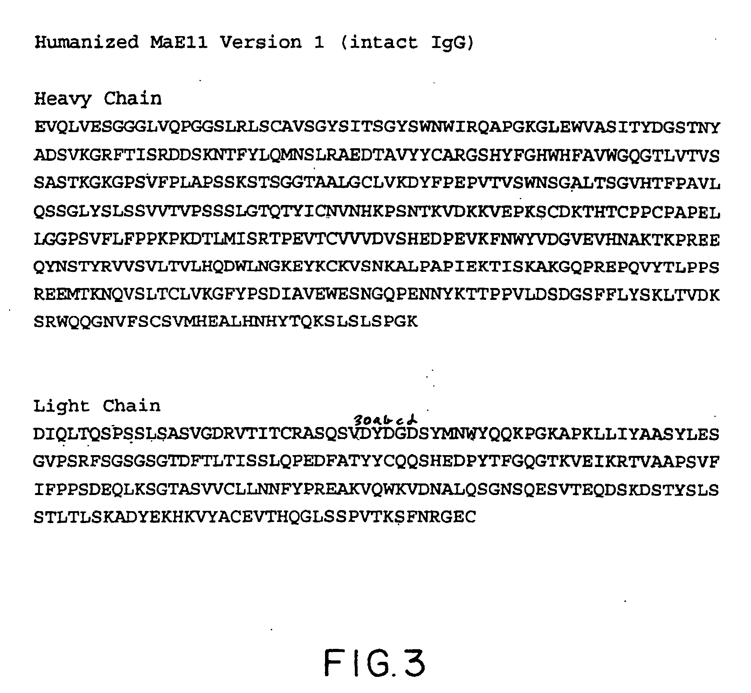

The characteristics possessed by the MAE11 antibody were preferred for therapeutic use. Since MAE11 bound to soluble IgE, bound to IgE bearing B cells, blocked IgE binding to the low and high affinity IgE receptor, inhibited *in vitro* IgE production and failed to bond to IgE coated basophils, it was chosen as the donor antibody for humanization. The recipient antibody was Kabat human kappa (light) subgroup I and human subgroup III heavy chain, although it will be understood that any other human antibody can be suitably employed. Surprisingly, optimal results were not obtained by simply substituting the murine CDRs in place of the CDRs in a recipient human antibody (Fig. 3; Table 4 *infra*). Instead, it was necessary to restore donor framework hydrophobic residues such as V_{H} 78, 48, 49, 63, 67, 69; 82 or 82c, or V_{L} 13, 19, 58, 78 or 104, in order to achieve a degree of inhibition of IgE binding similar to that of the donor antibody. While these residues function to establish the conformation of CDRs, they generally are not exposed to the exterior of the antibody so use of the murine residues should not exert a significant impact on immunogenicity. Other non-CDR residues exerting an effect on binding included V_{H}60, 61, 37, 24, and V_{H}50, 52, 58 and 95 (non-CDR by Chothia), and V_{L}4, V_{L}33 (non-CDR by Chothia) and V_{L}53 (non-CDR by Chothia). The human framework hydrophobic residues generally are substituted with other hydrophobic residues (especially those from the donor antibody) such as valine, isoleucine, leucine, phenylalanine or methionine. The remaining non-CDR residues are substituted with any other amino acid residue, but again preferably the murine residue found at the analogous site.

In general, the character of the anti-IgE antibody is improved by substituting, deleting or inserting a residue at or adjacent to V_{L} sites 30, 30b, 30d, 33, 55, 57, 58, 78, 93, 94, or 104 residues 30, 30a, 30b, 30c, 30d are identified with reference to the sequence DYDGD in the light chain sequence depicted in Figure 3) and/or V_{H} residues 24, 37, 48, 49, 54, 57, 60, 61, 63, 65, 67, 69, 78, 82, 82c, 97, 100a or 100c.

Position V_{H}-78 is most preferably substituted with phenylalanine. However, it also is substituted with leucine, valine, isoleucine, methionine, alanine or any other residue which results in an improvement in the characteristics of the antibody (see infra).

Position V_{H}-60 is most preferably substituted with asparagine, although substitution with glutamine, histidine, lysine, arginine or any other residue which improves the characteristics of the antibody shall fall within the scope of this invention.

Position V_{H}-61 is most preferably substituted with proline, although glycine, alanine, valine, leucine, isoleucine or any other residue which results in an improvement in the characteristics of the antibody also is suitable.

CDR residues were imported from the donor MaE11. These included four inserts in V_{L1} 30a-30d as well as 91-94 (V_{L3}), V_{H1} 27-29, 29a, 31, 33 and 34, V_{H2}53-55, and V_{H3}97-101. V_{L} 30, 30b or 30d as well as V_{H}97, 100a or 100c, are important in conferring on the CDR ability to bind IgE.

V_{H} positions 97, 100a and 100c in humae11 (humanized Mae11) are all histidine, and 2 are arginine in MaE15. These residues are important in IgE binding. One, two or three of these are modified by substitution with basic residues, particularly lysine or arginine, but also with alanine, glycine, valine, isoleucine, serine, threonine, aspartic acid, glutamic acid, asparagine, glutamine, methionine, phenylalanine, tyrosine, tryptophan or proline.

V_{L} positions 30, 30b and 30d of humae11 also are important for IgE binding. In humae11 each of these positions are occupied by the acidic residue, aspartic acid. They are substituted in other embodiments by glutamic acid, but also may be substituted with alanine, glycine, valine, isoleucine, serine, threonine, asparagine, glutamine, methionine, phenylalanine, tyrosine, tryptophan or proline. It is within the scope of this invention to reverse the charges on positions V_{L} 30, 30b and 30d with those on V_{H} 97, 100a and 100c, e.g. by employing aspartic acid residues in the three V_{H} sites (2 in the case of humanized MaE15) and histidine in the three V_{L} sites.

Residues also may be inserted adjacent to V_{H} positions 97, 100a, 100c, 61 or 61, or V_{L} residues at positions 30, 30b, 30d or 78. Inserted residues generally will be of like kind, e.g. an acid residue would be inserted adjacent to V_{L}-30, 30b or 30d, while a basic residue is inserted adjacent to V_{H}-97, 100 or 100c. The residues at these sites also may be deleted.

Humanized IgE-monovalent antibodies also are included within the scope of this invention. In this instance humanization extends to the anti-IgE arm as well, if necessary, to the remaining arm(s). Non-IgE binding arms of course can originate from human antibodies and in such case will not require humanization.

The foregoing variations are made by introducing mutations into the DNA encoding the precursor form of the antibody and expressing the DNA in recombinant cell culture or the like. This is accomplished by conventional methods of site directed mutagenesis. The variants then are screened for the desired character in assays conventional *per se*. In the case of anti-huIgE, desired character includes increasing the antibody affinity for hulgE, increasing its capacity and specificity for FCEL bound IgE, increasing the concentration of antibody required to stimulate histamine release from mast cells or basophils, reducing immunogenicity in humans, and other improvements apparent to the ordinary artisan. Optimizing these characteristics frequently will require balancing one improvement against another and therefore is a matter of judgment and is dependent upon the performance parameters dictated by the use intended for the antibody.

It is preferable to use a human IgG1 (or other complement fixing antibody) as the recipient immunoglobulin for humanization, although hu IgG2, IgG3, IgG4, IgE, IgM, IgD or IgA also can be used as recipient. Preferably the recipient is a complement fixing IgG antibody or an IgG antibody capable of participating in ADCC.

### Therapeutic, Diagnostic and Preparatory Uses

The anti-IgE antibodies herein are useful in identifying IgE amino acid sequence variants in which the FCEL or FCEH-binding domains have been modified. Candidate FCEL or FCEH-specific polypeptides are incubated with these antibodies, and analogues to which these antibodies fail to bind are selected for further evaluation, e.g., determination, respectively of their FCEH and FCEL receptor binding characteristics. Any antibody, whether of murine, human, or another animal species in origin, or a variant thereof such as the humanized immunoglobulins described above, which has the epitopic specificity of any of antibodies MAE10 - MAE17 (especially MAE11/15, MAE13 or MAE17) will be equally acceptable. Such antibodies are easily identified by immunizing a suitable animal or using an *in vitro* Fv selection system, e.g. phagemid, with IgE of the appropriate animal origin and screening the animals or products for antibodies having the ability to compete for IgE with MAE11/15, 13, 17 or other antibodies which map to substantially the same epitopic site(s) as those described herein. As noted, the antibodies desirably are monovalent for FCEL- bound IgE when employed therapeutically. They may be bivalent and/or bispecific when used to purify IgE from plasma, serum or recombinant cell culture.

The anti-IgE antibodies (especially those with reduced immunogenicity) are useful in therapies for the treatment or prophylaxis of allergies.

The antibodies typically are administered to a patient who is known to be sensitized to an allergen, preferably prior to an acute allergic response. The dosages and administration route will depend upon the accessory functionalities accompanying the antibody (e.g. cytotoxic agents, immunoglobulin effector functions, etc.), the condition of the patient (including the population of B cells or mast cells and basophils), the half-life of the antibody, the affinity of the antibody for its receptor and other parameters known to the clinician. As a general guide, one will determine from blood tests the amount of target cells circulating in the patient and determine the amount of antibody to displace or effectively compete with endogenous IgE taking into account the population of FCEH receptors as well as the half life and affinity of the antibody for IgE.

Therapeutic polypeptides are administered by intravenous intrapulmonary, intraperitoneal subcutaneous or other suitable routes. Preferably the polypeptides are administered s.c. or i.v. over a period of about from 1 to 14 days as required. In the case of suppressing IgE synthesis one would determine the amount of anti-IgE antibody needed to inhibit, suppress or kill a substantial portion of the IgE-secreting B cell population. Inhibition or suppression of the B cell population includes either or both of reductions in IgE secretion and attenuation of the total number of IgE secreting B cells. Candidate doses are readily determined by the use of *in vitro* cell cultures or animal models.

Therapy of allergic disorders with anti- FCEL bound IgE optionally is accomplished with other known therapies for allergies. These include administration of gamma interferon, allergen desensitization, reduction in exposure to allergen, treatment with antihistamines and the like.

### EXAMPLE 1

### Preparation of monoclonal antibodies to IgE

Eight monoclonal antibodies with the ability to block the binding of IgE to the FCEH were used. These monoclonal antibodies, referred to as MAE10 - MAE17, were made in the following manner. Purified human IgE was prepared from supernatants of U266B1 cells (ATCC TIB 196) using affinity chromatography on a previously isolated anti-IgE antibody (Genentech MAE1, although other anti-huIgE antibodies are equally useful). For MAE12, five BALB/c female mice, age six weeks, were immunized in their foot pads with 10 µg of the purified IgE in Ribi's adjuvant. Subsequent injections were done in the same manner one and three weeks after the initial immunizations. Three days after the final injection, the inguinal and popliteal lymph nodes were removed and pooled, and a single cell suspension was made by passing the tissue through steel gauze. For MAE14, MAE15, and MAE13 the immunizations were done in a similar manner except that for MAE13 30 µg of IgE per injection were used and IgE 315-547 was used as a prefusion boost; for MAE14 and MAE15 five injections of 50 µg each were used; and the IgE immunogen for MAE17 was IgE 315-547. For MAE10 and MAE11, injections were given subcutaneously in two doses of 100 µg and a final booster of 50 µg, and spleen cells were used for the fusions. The cells were fused at a 4:1 ratio with mouse myeloma P3X63-Ag8.653 (ATCC CRL 1580) in high glucose (DMEM) containing 50% w/v polyethylene glycol 4000.

Fused cells were plated at a density of 2x10⁵ per well in 96 well tissue culture plates. After 24 hours HAT selective medium (hypoxanthine/aminopterin/thymidine, Sigma Chemical Company, # H0262) was added. Of 1440 wells plated, 365 contained growing cells after HAT selection.

Fifteen days after the fusion, supernatants were tested for the presence of antibodies specific for human IgE using an enzyme-linked immunosorbent assay (ELISA). The ELISA was performed as follows, with all incubations done at room temperature. Test plates (Nunc Immunoplate) were coated for 2 hours with rat anti-mouse IgG (Boehringer Mannheim, # 605-500) at 1 µg/ml in 50 Mm sodium carbonate buffer, Ph 9.6, then blocked with 0.5% bovine serum albumin in phosphate buffered saline (PBS) for 30 minutes, then washed four times with PBS containing 0.05% Tween 20 (PBST). Test supernatants were added and incubated two hours with shaking, then washed four times with PBST. Human IgE (purified from U266 cells as described above) was added at 0.5 µg/ml and incubated for one hour with shaking, then washed four times in PBST. Horseradish peroxidase conjugated goat anti-human IgE (Kirkegaard & Perry Labs, # 14-10-04, 0.5 mg/ml) was added at a 1:2500 dilution and incubated for one hour, then washed four times with PBST. The plates were developed by adding 100 µl/well of a solution containing 10 mg. of o-phenylenediamine dihydrochloride (Sigma Chemical Company # P8287) and 10 µl of a 30% hydrogen peroxide solution in 25 ml of phosphate citrate buffer Ph 5.0, and incubating for 15 minutes. The reaction was stopped by adding 100 µl/well of 2.5 M sulfuric acid. Data was obtained by reading the plates in an automated ELISA plate reader at an absorbance of 490 nm. For MAE12, 365 supernatants were tested and 100 were specific for human IgE. Similar frequencies of IgE specificity were obtained when screening for the other antibodies. All of the monoclonal antibodies described herein were of the IgG1 isotype except for MAE17, which was IgG2b, and MAE14, which was IgG2a.

Each of the IgE specific antibodies was further tested in cell-based and plate assays to select for antibodies which bound to IgE in such a way as to inhibit IgE binding to FCEH and which are not capable of binding to FCEH-bound IgE. The results of these assays are set forth in Table 1a and Table 1b below.

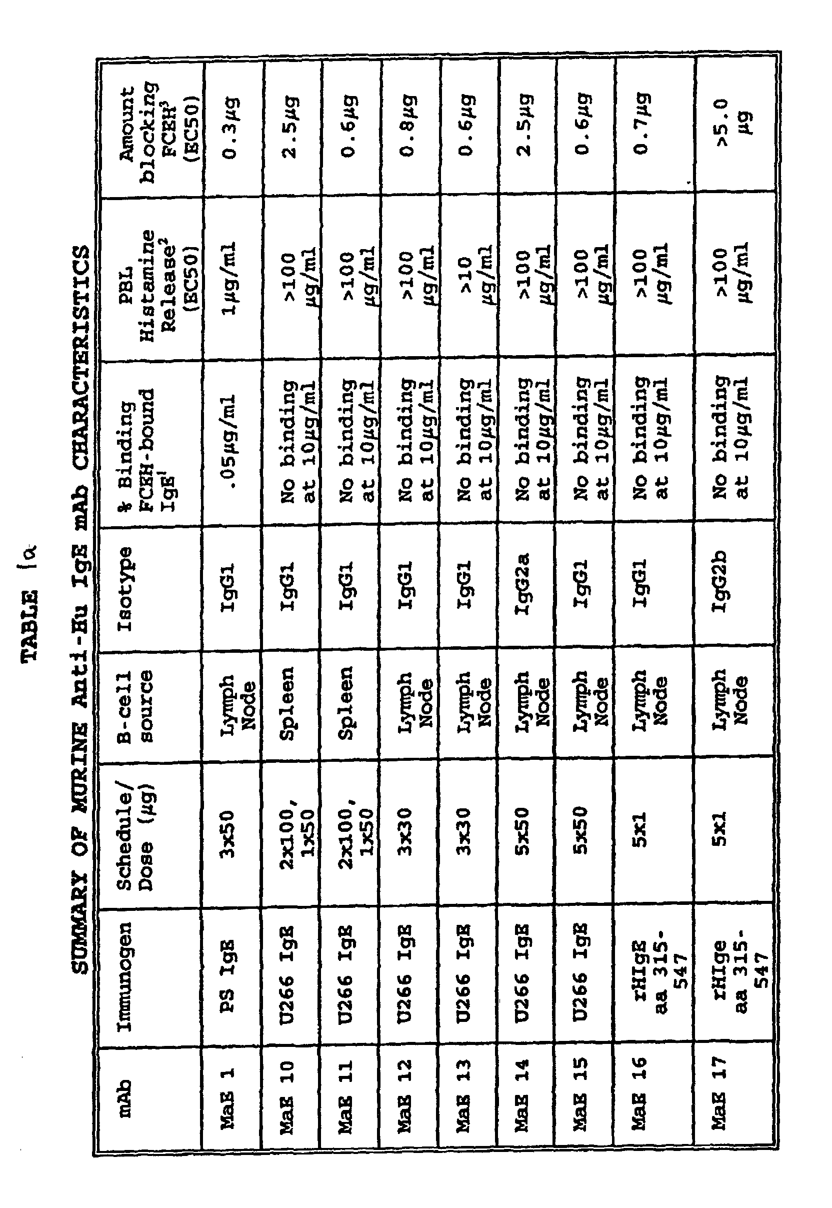

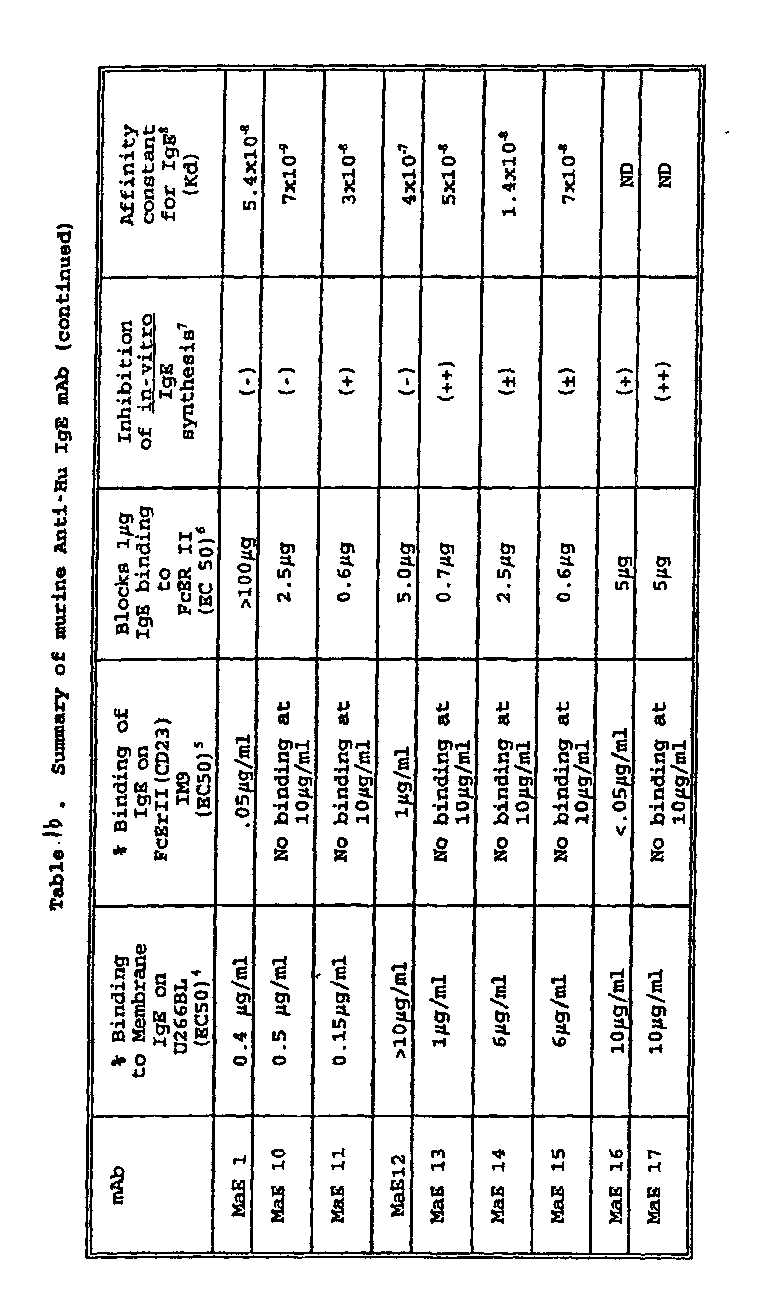

### 1. FACS based assays for analysis of murine anti-human IgE monoclonals. Screen of murine anti-human IgE monoclonal binding to IgE on CHO 3D10 (FcERI alpha +)

a. CHO 3D10 cells (FcERI alpha chain stable transfectant; Hakimi *et al.,* J. Biol. Chem. **265**:22079) at 5 x 10⁵ cells per sample are incubated with U266 IgE standard (lot no. 13068-46) at 10µg/ml in 100 µl FACS buffer (0.1% BSA 10mN sodium azide in PBS pH 7.4) for 30 minutes at 4°C followed by one wash with FACS buffer. The amount of IgE binding is determined by incubating an aliquot of IgE loaded cells with a polyclonal FITC conjugated rabbit anti-human IgG (Accurate Chem. Co. AXL-475F, lot no 16) at 50 µg/ml for 30 minutes at 4°C followed by three washes with FACS buffer.
b. IgE loaded cells are incubated with 100µl of murine anti-human IgE hybridoma supernatant (murine IgG concentration ranging from 1 to 20 µg/ml) for 30 min. at 4°C followed by one wash with FACS buffer. A Genentech monoclonal anti-human IgE (MAE1) at 10µg/ml is used as a positive control for binding. Genentech monoclonal (MAD 6P) which does not recognize IgE is used at 10µg/ml as a negative control.
c. Monoclonal binding to human IgE on CHO cells is detected by incubating cells with 20 µg/ml FITC-conjugated, affinity purified F(ab) 2 Goat anti-mouse IgG (Organon Teknica cat. no. 10711-0081) for 30 minutes at 4°C followed by three washes with FACS buffer. Cells are added to 400µl buffer contain 2 µg/ml propidium iodide (Sigma cat no. P4170) to stain dead cells.
d. Cells are analyzed on a Becton Dickinson FACSCAN flow cytometer. Forward light scatter and 90 degree side scatter gates are set to analyze a homogeneous population of cells. Dead cells which stain with propidium iodide are excluded from analysis. Hybridoma supernatants which do not bind IgE on CHO 3D10 cells were considered candidates for further screening.

### 2. Histamine release from peripheral blood basophils:

Heparinized blood was obtained from normal donors and diluted 1:4 in a modified Tyrodes buffer (25mM tris, 150mM NaCl, 10 mM CaCl₂,MgCl₂, 0.3 mg/ml HSA, pH 7.35) then incubated with 1nM human IgE (ND) at 4°C for 60 minutes. Cells were then added to Tyrodes buffer containing either the murine monoclonal anti-IgE Abs (10 mg/ml) or a polyclonal anti-human antiserum as the positive control, and incubated at 37°C for 30 minutes. Cells were pelleted, histamine in supernatants was acetylated and histamine content was determined using an RIA kit (AMAC, Inc. Wesbrook, Main). Total histamine was determined from cells subjected to several rounds of freezed thawing. Percent histamine release was calculated as nM histamine content in supernatant - nM histamine spontaneously released divided by nM total histamine in the sample.

### 3. Blocking of Fitc conjugated IgE binding to FcERI alpha chain.

The effect of the antibodies on IgE binding was studied by preincubating Fitc labelled IgE with the various Mae antibodies at 37° C for 30 minutes in PBS containing 0.1% BSA and 10mM Sodium Azide pH 7.4, then incubating the complex with 5 x 10⁵ 3D10 cells at 4°C for 30 minutes. The cells were then washed three times and mean channel fluorescence at 475 nM was measured. A murine anti-human IgE mAb (Mae1) which does not block IgE binding to the FcERI alpha chain was used as a control.

### 4. Analysis of murine anti-human IgE binding to membrane IgE positive B cell U266

a. U266 B1 cells (membrane IgE +) are cultured in base medium supplemented with 15% head inactivated fetal calf serum (Hyclone cat no. A-1111-L), penicillin, streptomycin (100 units/ml) and L-glutamine (2mM).
b. Cells (5x10⁵/aliquot) are incubated in 100µl FACS buffer containing murine anti-Human IgE monoclonals at 10, 5, 1, 0.5, and 0.1µg/ml for 30 minutes on ice in 96 well round bottom microtiter plates followed by two washes with FACS buffer. The Genentech monoclonal MAE1 is used as a positive control.
c. Cells are incubated in 100µl FACS buffer containing 50µg/ml (1:20 stock) FITC conjugated F(ab') 2 affinity purified goat anti-mouse IgG (Organon Teknika Cat. no. 1711-0084) for. 30 minutes on ice followed by three washes with FACS buffer. Cells are added to 400 µl FACS buffer containing propidium iodide at 2 µg/ml to stain dead cells.

### 5. FACS based binding assays to FcERII(CD23+) B cell IM9

a. FACS analysis of IgE binding to FcERII(CD23) (+) B cell line IM9. The IM9 human B cell myeloma ATCC CCL 159. (Ann. N.Y. Acad. Sci., **190**:221-234 [1972]) was maintained in GIF base medium with 10% heat inactivated fetal bovine serum, penicillin, streptomycin (100 units/ml) and L-glutamine (2mM).
b. Cells (5 x 10⁵ aliquot) were incubated in 100µl of FACS buffer containing U266 IgE standard at 2µg/ml for 30 minutes at 4°C in 96 well microtiter plates followed by 2 washes with FACS buffer. As a control, cells were incubated in buffer alone or buffer containing 2µg/ml human IgG1 (Behring Diagnostics Cat. no. 400112, lot no. 801024).
c. Cells were then incubated with murine anti-human IgE monoclonals at 0.1 to 10µg/ml for 30 minutes on ice. Genentech monoclonal MAE1 was used as a positive control.
d. Cells were incubated in 100µl FACS buffer containing FITC conjugated F(ab¹)2 goat anti-mouse IgG at 50 µg/ml (Organon Teknika Ca #1711-0084) for 30 minutes at 4°C followed by 3 washes with FACS buffer.
e. Cells were added to 400µl buffer containing propidium iodide at 2µg/ml to stain dead cells.
f. Cells were analyzed on a Becton Dickinson FACSCAN flow cytometer. Forward light scatter and 90 degree side scatter gates were set to analyze a homogeneous population of cells and dead cells which stained with propidium iodide were excluded from analysis. FITC positive cells (IgE binding) were analyzed relative to cells stained with FITC rabbit anti-Human IgE alone.
g. As a positive control to determine the level of CD 23 on the surface of IM9 cells in each experiment, an aliquot of cells was stained with Becton Dickinson murine monoclonal Leu 20 (anti-CD23) at 10µg/ml for 30 minutes at 4°C followed by 2 washes. The cells were then incubated with FITC conjugated f(ab') 2 affinity purified goat anti-murine IgG at 50µg/ml.

### 6. Antibody blocking of Fitc conjugated IgE binding to the low affinity IgE receptor.

The binding of 40 nM FITC labelled IgE to the low affinity IgE receptor (CD23) expressed on the B lymphoblast cell IM-9 was analyzed by flow cytometry on a FACSCAN flow cytometer. The effect of the antibodies on Fitc IgE binding was studied by preincubating Fitc IgE with the murine anti-human antibodies at 0.1 to 10µg/ml. chimera at 37°C for 30 minutes in PBS containing 0.1% BSA and 10mM Sodium Azide pH 7.4, then incubating the complex with 5 x 10⁵ cells at 4°C for 30 minutes. The cells were then washed three times and mean channel fluorescence at 475 nM was measured.

### 7. IgE In Vitro Assay Protocol

a. Peripheral blood mononuclear cells were separated from normal donors.
b. Cells were washed extensively with phosphate buffered saline to remove as many platelets as possible.
c. Mononuclear cells were counted and resuspend in media at 1x10⁶ cells/ml. (Media=DMEM + pen/strep + 15% horse serum + IL-2 (25U/ml) + IL-4 (20ng/ml)).
d. Antibodies were added at appropriate concentrations on day 0, 5, and 8.
e. Cultures were incubated in 24 well Falcon tissue culture plates for 14 days.
f. On day 14 supernatants were removed and assayed for IgE concentrations by an IgE specific ELISA protocol.

### 8. Affinity constant (kd) of murine mAb for human IgE was determined by equilibrium binding (Scatchard analysis as follows:

a. IgE (ND and PS allotypes were iodinated by the chloramine T method and separated from free ¹²⁵I Na with a PD10 sephadex G25 column (Pharmacia cat. no. 17-0851-01) in RIA buffer:PBS, 0.5% bovine serum albumin (Sigma cat. no. A-7888), 0.05% Tween 20 (Sigma cat. no. P-1379), 0.01% thimerosal (Sigma cat. no. T-5125), pH 7.4. Approximately 78-95% of the post column counts were precipitated with 50% trichloroacetic acid and specific activity of iodinated IgE preparations ranged from 1.6 to 13 µCi/µg assuming 70% counting efficiency.
b. A fixed concentration of ¹²⁵I IgE (approximately 5 x 10⁴ cpm) was added to varying concentrations of unlabelled IgE (1 to 200 nM) in a final volume of 0.1ml RIA buffer in 12x75mm polypropylene test tubes. Murine anti-human IgE mABs (20nM final concentration) in 0.1 ml RIA buffer were then added for a final assay volume of 0.2ml.
c. Samples were incubated 16-18 hours at 25°C with continuous agitation.
d. Bound and free ¹²⁵I IgE was separated by the addition of a 0.3 ml mixture of affinity purified goat anti-mouse IgG (Boehringer Mannheim cat. no. 605 208) coupled to CN Br activated Sepharose 4B (cat no. 17-0430-01) and carrier protein A sepharose (Repligen cat. no. IPA 300) in RIA buffer and incubated 1 to 2 hours at 25°C with continuous agitation. RIA buffer (1ml) was then added, and tubes were centrifuged 5 min. 400 xg. Samples were counted to determine total counts. Supernatants were aspirated with a finely drawn pasteur pipet, samples were recounted and bound versus free counts were calculated.
e. Scatchard analysis was performed utilizing a Fortran program (scanplot) based on the Ligand program written by P. Munson at NIH. Scatplot uses a mass action equation fitting bound as a function of total using the Rodbard type regression analysis.

### EXAMPLE 2

### Preparation of Variant IgE

Based on the model of IgE Fc by Padlan & Davies (Mol. Immunol. 23:1063 (1986), which is based on the crystal structure of human IgG1 Fc (Deisenhofer, Biochem. **20:**2361-2370 [1981]), a series of mutants were designed which could be used to test the binding of human IgE to its receptors. These mutants are designated Emut 1-13, and are listed in Table 2 below. The Fcε3 domain is comprised of seven β-strands which form a β-sheet structure representative of all immunoglobulin domains; there are six loops which connect these seven β-strands. We refer to these loops by the 2 β-strands they connect, e.g. loop AB connects β-stands A and B. We have constructed mutants of human IgE in which we have substituted five of the Fcε3 domain loops with their counterparts from human IgG1 (Table 2, 1-5). The sixth loop contains the glycosylation site in both IgE and IgG and hence was not altered. One mutant, (Table 2, 6), was made by exchanging human Fcε3 β-strand D with its human IgG1 Fcgamma2 counterpart. Seven additional mutants, (Table 2, 7-13), consisted of the substitution of Ala residues into Fcε3 β-strands and a loop in Fcε2.

A human IgE gene was cloned from U266, a publicly available cell line. The gene was cloned into a previously described phagemid vector containing the human cytomegalovirus enhancer and promoter, a 5' intron and sv40 polyadenylation signal (Gorman *et al.,* DNA and Prot. Eng. Techn., **2**:3-10 [1990]). Mutagenesis was performed by the Kunkel method (T.A. Kunkel, Proc. Natl. Acad. Sci. USA, **82**:488-492 (1985) using buffers and enzymes supplied with the BioRad Muta-gene phagemid *in vitro* mutagenesis kit, together with oligonucleotides encoding the human IgG1 sequences shown in Table 2 below. Sequences of the mutant IgE DNAs were checked only at the site of mutation using ³⁵S dideoxy sequencing

**TABLE 2**

| **Mutant** | **Kabat Residue No. (Structure)**^{**1**} | **Human IgeE** **Fcε3 Seq.** | **Human IgG1** **Fcγ2 Seq.** |
|---|---|---|---|
| 1 | 377-385 (lAB) | FDLFIRKS (SEQ.ID NO. 10) | KDTLMISRT (SEQ.ID NO. 11) |
| 2 | 396-401 (lBC) | APSKGT (SEQ.ID NO. 12) | SHEDPQ (SEQ.ID NO. 13) |
| 3 | 407-420 (ICD) | SRASGKPVNHS (SEQ.ID NO. 14) | YVDGVQVHNAK (SEQ.ID NO. 15) |
| 4 | 444-453 (lEF) | GTRDWIEGET (SEQ.ID NO. 16) | LHQDWLDGKE (SEQ.ID NO 17) |
| 5 | 465-469 (lFG) | RALM (SEQ.ID NO. 18) | APIE (SEQ.ID NO. 19) |
| 6 | 423-428 (βD) | KEEKQR (SEQ.ID NO. 20) | PREQQY (SEQ.ID NO. 21) |
| 7 | 383-385 (lAB) | RKS | [AAA]² |
| 8 | 387, 389 (βB) | T(I)T | [A(I)A]² |
| 9 | 403, 405 (βC) | N(L)T | [A(L)A]² |
| 10 | 438-440 (βE) | T(S)T | [A(S)A]² |
| 11 | 455, 457, 459 (βF) | Q(C)R(V)T (SEQ.ID NO. 22) | [A(C)A(V)A]² (SEQ.ID NO. 23) |
| 12 | 471, 473 (βG) | S(T)T | [A(T)A]² |
| 13 | 329-331, 334-336 | QKH(WL)SDR (SEQ.ID NO. 24) | [AAA(WL)AAA]² (SEQ.ID NO. 25) |

| | | | |
|---|---|---|---|
| ¹loop = l B-strand = β | | | |
| ²Sequences in brackets are from mutants in which alanine residues rather than IgG sequences were used to replace the IgE target sequence. Residues in parentheses were not altered in these mutants. | | | |

The mutant IgEs were transiently expressed in human embryonic kidney 293 cells (Gorman et al., *supra*), purified on a mouse anti-human IgE antibody affinity column and samples run using SDS-PAGE to ascertain that the mutant proteins were of the proper molecular weight.

### EXAMPLE 3

### Soluble FCEH binding assay

This assay is a sequential inhibition ELISA which measures binding to the FCEH only. In this assay, a monoclonal antibody against the FCEH is coated onto ELISA plates at a concentration of 1 µg/ml in 50 mM sodium carbonate pH 9.6 for two hours at room temperature, and blocked for two hours with PBS containing 0.5% bovine serum albumin (PBSA), then washed three times with ELISA wash buffer (0.05% Tween 20 in PBS). Recombinantly produced soluble FCEH is added at a concentration of 50 units/ml and incubated for one hour, then washed five times in ELISA wash buffer. Mutant IgE samples are then added to the wells and incubated for one to two hours. The excess mutant IgE is removed by aspiration, and biotinylated IgE is then added at 50 ng/ml for 15 minutes followed by five washes with ELISA wash buffer. Streptavidin conjugated to horseradish peroxidase (Sigma Chemical Company #S5512) was added at a 1:5000 dilution for 15 minutes, then washed three times with ELISA wash buffer. Color was developed with a tetramethyl benzidine peroxidase substrate system (Kirkegaard &. Perry Labs # 50-76-00, Lot. no. NA 18) for seven minutes at 25° C. The reaction was stopped by the addition of 1 M HCl. The ability of the mutant IgE to bind the FCEH is assessed by the degree to which the biotinylated IgE is prevented from binding. This assay is designed to test for any FCEH binding by the mutant IgE and is not meant to determine the affinity of the mutant for the FCEH relative to native IgE.

### FACS based binding assays for U266 IgE mutants

Tissue culture supernatants from 293s cells transfected with U266 IgE cDNA were harvested at either 48 or 96 hours post transfection. Tissue culture supernatants were concentrated 5-X with Amicon Centriprep 30® centrifugal concentrators (30,000 MW cutoff). Concentrated supernatants were passed through a mouse monoclonal anti-U266 IgE affinity column (Genentech MAE1 coupled to CnBr-Sepharose). U266 IgE was eluted from the column with 3.0 M potassium cyanate in 50 mM tris buffer Ph 7.8. Eluate fractions containing protein as determined by 0.D.280 nm were pooled and placed in Amicon Centricon 30® concentrators. Eluate buffer was exchanged for PBS by passing multiple volumes of PBS through the concentrator. The final volume of affinity purified supernatant ranged from 0.5-1 ml. Structural integrity of recombinant IgE mutants was analyzed on 1-12% SDS PAGE gels and compared with U266 IgE standard obtained from the U266 cell line. Mutants were also analyzed for the ability to bind to a series of monoclonal and IgE antibodies to further ascertain proper folding and structural identity with native IgE. The concentration of immunoreactive IgE for each IgE mutant was determined by a human IgE capture ELISA as follows. Nunc Immunoplate Maxisorp® plates (Nunc # 4-39451) were coated overnight at 4° C with a Genentech murine IgGl anti-U266 IgE (MAE1) at 1 µg/ml in coat buffer (50 mM sodium carbonate buffer pH 9.6 ). Coat antibody was removed by three washes with ELISA wash buffer (0.05% Tween 20 (US Biochemical Corporation # 20605) in PBS). Non-specific sites were blocked with ELISA diluent buffer (50 mM tris buffered saline containing 0.5% BSA (Sigma Chemical Company # A-7888), 0.05% Tween 20 and 2 mM EDTA) for two hours at 25° C on an orbital shaker. Diluent buffer was removed with 3 washes of ELISA wash buffer. Serial two-fold dilutions of IgE mutants in ELISA diluent buffer were added to the plate. U266 IgE standard (lot 13068-46) was added at 1000, 500, 250, 125, 62.5, 31.3, and 15.6 ng/ml in duplicate as standards. Samples and standard were incubated two hours at 25° C followed by three washes with ELISA wash buffer. IgE was detected with HRP conjugated Sheep anti-human IgE (ICN # N060-050-1) at 1:8000 in ELISA diluent buffer for 90 min. at 25° C followed by 3 washes with ELISA wash buffer. HRP conjugate was developed with a tetramethyl benzidine peroxidase substrate system (Kirkegaard & Perry Labs. # 5076-00, Lot. no. NA 18) for 7 minutes at 25° C. The reaction was stopped by the addition of 1 M HCl. The reaction product was analyzed with a dual wavelength spectrophotometer at 450 nm minus absorption at 570nm. The U266 IgE standards were used to generate a standard curve and IgE concentrations of the sample were extrapolated by non-parametric linear regression analysis.

FcERI alpha (+) CHO 3D10 (FCEH expressing) and FcERII(CD23) (+) IM9 (FCEL expressing) B cell lines were used for the binding assays. The stably transfected CHO (duk -) cell clone 3D10 (JBC **265**, 22079-22081, 1990) was maintained in Iscove's modified Dulbecco's media supplemented with 10% heat inactivated fetal calf serum, 80 µg(ml gentamicin sulfate and 5 X 10 ⁻⁷M methotrexate. The IM9 human B cell myeloma ATCC CCL 159. (Ann. N.Y. Acad. Sci. 190:221-234, 1972) was maintained in GIF base medium with 10% heat inactivated fetal bovine serum, penicillin, streptomycin (100 units/ml) and L-glutamine (2mM). As a positive control to determine the level of CD23 on the surface of IM9 cells in each experiment, an aliquot of cells was stained with Becton Dickinson murine monoclonal Leu 20 (anti-CD23) at 10 µg/ml for 30 minutes at 4° C followed by two washes in FACS buffer. The cells were then incubated with FITC conjugated F(ab')2 affinity purified goat anti-murine IgG at 5 µg/ml. Adherent CHO3D10 cells were removed from tissue culture dishes by incubation with 10 mM EDTA in PBS for 2 minutes at 37°C. Cells were counted, then resuspended in FACS buffer (0.1% BSA, 10 mM Na azide in PBS pH 7.4 ) at a concentration of 5x10⁶/m]. CHO3D10 and Im9 cells (5 x 10⁵/aliquot) were incubated in 100 µl of FACS buffer containing U266 IgE standard or IgE mutants at 2µg/ml for 30 minutes at 4° C in 96 well microtiter plates followed by two washes with FACS buffer. As a control, cells were incubated in buffer alone or buffer containing 2 µg/ml human IgG1 (Behring Diagnostics # 400112, lot no. 801024). Cells were then incubated in 100 µl FACS buffer containing FITC conjugated rabbit anti-human IgE at 20 µg/ml (Accurate Chem. Co. # AXL 475F, lot.no. 040A) for 30 minutes at 4° C followed by 3 washes with FACS buffer. 400 µl of buffer containing propidium iodide at 2µg/ml was added to the cell suspension to stain dead cells. Cells were analyzed on a Becton Dickinson FACSCAN flow cytometer. Forward light scatter and 90 degree side scatter gates were set to analyze a homogeneous population of cells and dead cells which stained with propidium iodide were excluded from analysis. FITC positive cells (IgE binding) were analyzed relative to cells stained with FITC rabbit anti-H IgE alone.

The foregoing assays were used to determine the ability of the example 2 IgE analogues to bind to FCEH and FCEL. The results are set forth in Table 3.

**TABLE 3**

| **BINDING OF IGE AND IGE ANALOGUES TO FCEH AND FCEL** | | | |
|---|---|---|---|
| **Sample/Mutant** | **Conc. (ug/ml)** | **FCEH alpha % CHO 3D10(+)** | **FCEL (CD23) % IM9 (+)** |
| U266 IgE | 10 | 90.3 | 92.5 |
| U266 IgE | 5 | 89.9 | 82.6 |
| U266 IgE | 0.5 | 59.6 | 4.6 |
| U266 IgE | 0.1 | 15.8 | 1.7 |
| 1 | 1.65¹ | 1.7 | 4.3 |
| 2 | 1.65 | 34.3 | 48.9 |
| 3 | 1.65 | 32.3 | 1.2 |
| 4 | 1.65 | 4.9 | 9.2 |
| 5 | 1.65 | 60.5 | 73.9 |
| 6 | 1.65 | 1.4 | 71.6 |
| 7 | 1.65 | 76.4 | 4.6 |
| 8 | 1.65 | 70.3 | 16.3 |
| 9 | 1.65 | 84.2 | 94.3 |
| 10 | 1.65 | 67.5 | 84.8 |
| 11 | 1.65 | 70.8 | 61.5 |
| 12 | 1.65 | 84.7 | 90.3 |
| 13 | 1.65 | 85.7 | 96.1 |
| dh 184 (+) | 1.65 | 83.8 | 21.1 |
| PA13² (control) | 10 | 1.3 | |

| | | | |
|---|---|---|---|
| ¹Values based on quantitative Elisa. U266 was used as the standard and murine anti-F_{c}, monoclonal antibody to capture. | | | |
| ²A CDR grafted human IgG. | | | |

Three mutant IgEs exhibited complete loss of binding to the FCEH receptor: mutants 1, 4 and 6. Mutant 6 altered β-strand D at the end of Fcε3 close to the Fcε2 domain. Mutants 1 and 4 involved alteration of two Fcε3 loops which are adjacent and near the Fcε4 domain. Note that mutant 7 is subset of mutant 1 in which the three C-terminal residues of loop AB have been changed to alanines (Table 2, 1 vs. 7). However, mutant 7 does not affect binding to FCEH. We interpret this to mean that either 1) FcεRI binds at least one of IgE residues 377-381 or 2) the extra residue in IgG1 loop AB (9 residues) substituted for IgE loop AB (8 residues) effected deformation of some adjacent binding determinant, possibly loop EF. That mutants 8 and 10 had no affect on FcεRI binding most likely means that the FCEH receptor does not protrude into the cavity bounded by loop AB and β-strand D.

Although mutant 4 had a Leu replacing Gly444 (Table 2), this should not affect the conformation of loop EF. Residue 444 is prior to the N-terminus of this α-helix. In addition, murine IgE has a Val at position 444 and rat IgE has an Asp. The two buried hydrophobic residues in the middle of the α-helix, W448 and I449, are retained in the substituted IgG1 loop (W448, L449) as is G451 which terminated the α-helix. Hence the conformation of loop EF should be similar in IgE and IgG1.

Mutants 2 and 3 exhibited decreased binding to FCEH. Since loop BC lies near β-strand D and loop CD is in the vicinity of loop EF, it is conceivable that one or two residues in loops BC and CD contact FCEH.

Five mutant IgEs exhibited loss of binding to the FCEL receptor: mutants 1, 3 4, 7 and 8. Mutants 1 and 4 were discussed above. Mutant 3 involved alteration of loop CD; in contrast to FCEH, loop CD evidently plays a major role in FCEL binding. Mutant 7, a subset of mutant 1 as discussed above, comprises the C-terminal portion of loop AB and is proximal to loop EF. Additionally, mutant 8 consists of replacement of two Thr residues (387,389) with Ala; these two residues are part of β-strand B which is at the bottom of the aforementioned cavity bounded by loop AB and β-strand D. Mutant 10 comprised a different two residues in this cavity (438,440) on *β-*strand E, which is adjacent to β-strand B. Since mutant 10 did not affect FCEL binding, we conclude that the FCEL receptor should have only a minimal incursion into cavity while the high affinity receptor does not intrude into the cavity.

In addition to a glycosylation site at Asn430 which corresponds to the glycosylation site in IgG Fc, human IgE contains another glycosylation site at Asn403. Mutant 9 converted Asn403 and Thr405 to alanines (Table 2). Loss of carbohydrate did not affect binding to either receptor.

Based on the information from mutants 1-13, we propose that FCEH and FCEL have binding sites on IgE Fc which are distinct but overlap. The low affinity receptor seems to interact with a relatively smaller portion of the IgE Fcε3 domain involving three adjacent loops: AB, CD and EF. In contrast, the high affinity receptor interacts with a larger portion of IgE Fcε3, which spans loop EF, β-strand D and, possibly, the N-terminal portion of loop AB. Portions of loops BC and CD in the vicinity of loop EP and β-strand D may also interact with FCEH. In addition, FCEL may protrude into the cavity bounded by loop AB and β-strand D whereas FCEH does not do so. Since we have not evaluated any mutants in FCε4 and only one in Fcε2 (mutant 13), it is possible that portions of these two domains play a role in IgE-receptor binding.

### EXAMPLE 4

### Preparation of Humanized MaE11

Residues were selected from MaE11 and inserted or substituted into a human Fab antibody background (V_{H} region Kabat subgroup III and V_{L} region kappa subgroup I). A first version, humae11v1 or version 1, is described in Table 4.

**TABLE 4.**

| Changes in V_{H} human subgroup III and V_{L} κ subgroup I (Kabat) consensus sequences for humanized MaE11 Version 1 | | | | | |
|---|---|---|---|---|---|
| Domain | hu Residue | Residue No. | V.1 | CDR by Kabat | CDR by Chothia |
| V_{L} | | | | | |
| | M | 4 | L | | |
| | insert | 30 a, b, c, d | YDGD (SEQ.ID. NO. 26) | L1 | L1 |
| | L* | 33 | M | L1 | |
| | S | 53 | Y | L2 | |
| | Y | 91 | S | L3 | L3 |
| | N | 92 | H | L3 | L3 |
| | S | 93 | E | L3 | L3 |
| | L | 94 | D | L3 | L3 |
| V_{H} | | | | | |
| | A | 24 | V | | |
| | F* | 27 | Y | H1 | H1 |
| | T | 28 | S | H1 | H1 |
| | F* | 29 | I | H1 | H1 |
| | insert | 29a | T | H1 | H1 |
| | D | 31 | G | H1 | H1 |
| | A | 33 | S | H1 | H1 |
| | M* | 34 | W | H1 | H1 |
| | V | 37 | I | | |
| | V | 50 | S | H2 | |
| | S | 52 | T | H2 | |
| | N | 53 | Y | H2 | H2 |
| | G | 54 | D | H2 | H2 |
| | S | 55 | G | H2 | H2 |
| | Y | 58 | N | H2 | |
| | L | 78 | F | | |
| | D | 95 | G | H3 | |
| | | 97-101 | All Changed to MaE11 Sequence | H3 | H3 |

| | | | | | |
|---|---|---|---|---|---|
| * These residues typically do not vary despite their position within CDRs. The remaining residues found in the KI and III CDR sequences (particularly the CDRs by Chothia structural analysis), will vary widely among recipient human antibodies. | | | | | |

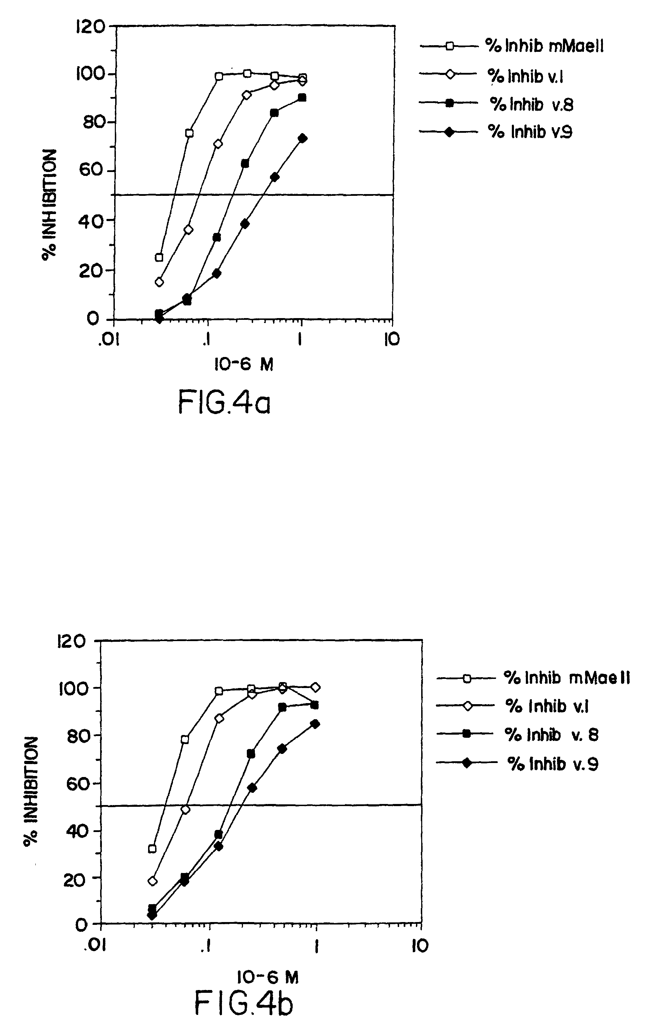

The affinity of version 1 was assayed and found to be about 100 times lower than that of the donor antibody Mae11 (see Figs. 4a and 4b). Therefore, further modifications in the sequence of version 1 were made as shown in Table 5. Determination was made of the ability of these further modifications to inhibit the binding of labelled hulgE to FCEH.

The 50% inhibition assays whose results are shown in Table 5 were conducted as follows:

A 96-well assay plate (Manufn Nunc.) was coated with 0.05 ml of the FcεRI alpha chain IgG1 chimeric receptor in 1 µg/ml coating buffer (50nmol carbonate/bicarbonate, pH 9.6). Assay was done for 12 hours at 4-8° C. The wells were aspirated and 250 µl blocking buffer (PBS--1% BSA pH 7.2) was added and incubated for one hour at 4°C. In a separate assay plate the samples and reference murine MaE11 antibody were titered from 200 µg/ml by 1 to 10-fold dilution with assay buffer (0.5% BSA, 0.05% Tween 20, PBS, pH 7.2) and an equal volume of 10ng/ml biotinylated IgE at 10ng/ml was added and the plate incubated for 2-3 hours at 25°C. The FcεRI-coated wells were washed three times with PBS-0.05% Tween20, and then 50 µl from the sample wells were transferred and incubated with agitation for 30 minutes at 25°C. 50 µl/well of streptavidin-HRP diluted 1:5000 in assay buffer was incubated for 15 minutes with agitation and then the plate was washed as before. 50 µl/well of Microwell peroxidase substrate (Kirkgaard & Parry Laboratories) was added and color was developed for 30 minutes. The reaction was stopped by adding an equal volume of 1 normal HCl and the adsorbance measured at 450nm. The concentration for 50% inhibition was calculated by plotting percent inhibition versus concentration of blocking antibody with a nonlinear 4-parameter curve-fit for each antibody using INPLOT.

**TABLE 5**

| **Humanized MaE11 Variants** | | | | | | |
|---|---|---|---|---|---|---|
| **Version [F(ab)- X]** | **Domain** | **Changes from F(ab)-Version 1** | **Purpose** | **Conc. at 50% inh. (ng/ml)* Mean** | **S.D. for prev. col.** | **F(ab)-X** **-------** **F(ab)-1** |
| 1 | - | - | - | 6083 | 1279 | 1.0 |
| 2 | V_{L} | L4M M33L | Packing; CDR-L1 | 9439 | 508 | 1.6 |
| 3 | V_{L} | E55G G57E | Sequence usually E55-X-G57 | 5799 | 523 | 1.0 |
| 4 | V_{H} | I37V | VL-VH interface | 8622 | 107 | 1.4 |
| 5 | V_{H} | V24A | Packing; CDR-H1 | 9387 | 733 | 1.6 |
| 6 | V_{H} | F78L | Packing; CDR-H1,H2 | 17537 | 4372 | 2.9 |
| 7 | V_{L} V_{H} | L4M R24K E55G G57E V24A I37V T57S A60N D61P V63L G65N F78L | remake version 1 to accomplish a direct exchange of CDR residues | > 100000 | | > 16.0# |
| 7a | V_{H} | As V.7 except V_{H} L78 is F | | 98000 | | 16.0 |
| 8 | V_{H} | A60N D61P | Extended Kabat CDR-H2 & A60N is at V_{L}-V_{H} interface | 1224 | 102 | 0.20 |
| 8a | V_{H} | As V.8 except V_{H} V63 is L and F67 is I | CDR-H2; packing of L63 and I67 | 416 | 66 | 0.07 |
| 8b | V_{H} | As V.8 except F67 is I | CDR-H2; packing of V63 and I67 | 501 | 84 | 0.08 |
| 1 | - | - | - | 6083 | 1279 | 1.0 |
| 9 | V_{L} | A13V V19A V58I L78V V104L | Repack Version 1 interior as in murine MaE11 | 842 | 130 | 0.14 |
| | V_{H} | V48M A49G A60N V63L F67I I69V M82L L82cA | | | | |
| 23 | V_{L} | L4M | Packing; CDR-L1 | 6770 | 349 | 1.1 |
| 10 | V_{L} | D30A D30Ab D30dA | CDR-L1 modification | > 100000 | | >16.0 |
| 11 | V_{L} | E93A D94A | CDR-L3 modification | 17456 | 7115 | 2.9 |
| 12 | V_{H} | D54A | CDR-H2 modification | 2066 | 174 | 0.34 |
| 13 | V_{H} | H97A H100aA H100cA | CDR-H3 modification | >100000 | | > 16.0 |
| 14 | V_{L} | D30A | CDR-L1 modification | 3452 | 183 | 0.57 |
| 15 | V_{L} | D30bA | CDR-L1 modification | 6384 | 367 | 1.0 |
| 16 | V_{L} | D30dA | CDR-L1 modification | >100000 | | > 16.0 |
| 17 | V_{H} | H97A | CDR-H3 modification | 19427 | 8360 | 3.2 |
| 18 | V_{H} | H100aA | CDR-H3 modification | 2713 | 174 | 0.45 |
| 19 | V_{H} | H100cA | CDR-H3 modification | 15846 | 8128 | 2.6 |

| | | | | | | |
|---|---|---|---|---|---|---|
| * Inhibition of fitc-IgE binding to FCEH (FcERI). Full length antibody and humanized fragment versions: mean and standard deviation of three assays. # A F(ab)-X / F(ab)-1 ratio of > 16 means that this variant exhibited no binding even at the highest F(ab) concentrations used. | | | | | | |

As can be seen from Table 5 and Figs. 4a and 4b, version 8 (in which human residues of version 1 at sites 60 and 61 in the light chain were replaced by their Mae11 counterparts) demonstrated substantially increased affinity. Further increases in affinity are seen in versions 8a and 8b, where one or two murine residues replaced human residues. Other increases, at least virtually to the level of Mae11, were accomplished by replacing hydrophobic human residues found in the interior of V_{H} and V_{H1} with their MaE11 counterparts, resulting in the variant designated version 9 (see Table 5 and Figs. 4a and 4b). Accordingly, the humanized antibodies of this invention will possess affinities ranging about from 0.1 to 100 times that of MAE11.

Table 6 explores the effects on FCEH affinity of various combinations of humanized maE11 IgG1 variants.

**Table 6.**

| **Humanized MaE11 IgG1 Variants** | | | | |
|---|---|---|---|---|
| **Variant** | **Conc. at 50% inh. (ng/ml) Mean*** | **S.D. from previous column*** | **Var. X** **-------** **IgL1H1** | **Var. X** **-------** **MaE11** |
| IgL1H1 | 7569 | 1042 | 1.0 | 16.9 |
| IgL1H8 | 3493 | 1264 | 0.46 | 7.8 |
| IgL9H9 | 1118 | 172 | 0.15 | 2.5 |
| IgL1H9 | 608 | 364 | 0.08 | 1.4 |
| IgL9H1 | 5273 | 2326 | 0.70 | 11.7 |
| IgL1H8b | 1449 | 226 | 0.19 | 3.2 |
| MaE11 | 449 | 53 | 0.06 | 1.0 |

| | | | | |
|---|---|---|---|---|
| * L1 = V_{L}, as in F(ab)-1 (human buried residues--not exposed to solvent); L9 = V_{L} as in F(ab)-9 (murine buried residues); H1 = V_{H} as in F(ab)-1 (human buried residues); H8 = V_{H} as in F(ab)-8 (F(ab)-1 with AlaH60Asn, AspH61Pro); H9 = V_{H} as in F(ab)-9 (murine buried residues); H8b = V_{H} as in F(ab)-8b (F(ab)-8 with PheH67Ile). | | | | |

### EXAMPLE 5

### Creation of IgE Mutants

IgE mutants (Table 7) were prepared to evalute their effect on binding to anti-IgE, especially MaE11, and to FcεRI and FcεRII. Some of the mutants were designed to substitute for a specific amino acid residue another residue with either similar or very different charge or size. The impact of these changes on receptor binding is reflected in the table below.

The receptor assays are performed substantially as follows:

A 96-well assay plate (Manufn Nunc.) was coated with 0.05 ml of FCεRI or RII IgG1 chimeric receptor in 1 µg/ml coating buffer (50nmol carbonate/bicarbonate, pH 9.6). Assay was done for 12 hours at 4-8° C. The wells were aspirated and 250 µl blocking buffer (PBS--1% BSA pH 7.2) was added and incubated for one hour at 4°C. In a separate assay plate the samples and reference murine MaE11 antibody were titered from 200 µg/ml by 1 to 10-fold dilution with assay buffer (0.5% BSA, 0.05% Tween 20, PBS, pH 7.2) and an equal volume of 10ng/ml biotinylated IgE at 10ng/ml was added and the plate incubated for 2-3 hours at 25°C. The FcεRI-coated wells were washed three times with PBS-0.05% Tween20, and then 50 µl from the sample wells were transferred and incubated with agitation for 30 minutes at 25°C. 50 µl/well of streptavidin-HRP diluted 1:5000 in assay buffer was incubated for 15 minutes with agitation and then the plate was washed as before. 50 µl/well of Microwell peroxidase substrate (Kirkgaard & Parry Laboratories) was added and color was developed for 30 minutes. The reaction was stopped by adding an equal volume of 1 normal HCl and the adsorbance measured at 450nm. The absorbance was plotted versus concentration of blocking antibody MaE11 and an inhibition standard curve was generated using INPLOT.

**Table 7.**

| **Amino acid sequences of IgE mutants** | | | | | |
|---|---|---|---|---|---|
| **Mutant** | **Kabat residue #** | **Human IgE Fce3 seq.** | **Mutant seq.** | **Fcε-RI*** | **FcεRII *** |
| Loop AB | | | | | |
| 1 | 377-385 | FDLFIRKS | KDTLMISRT | - | - |
| | | (SEQ.ID.27) | (SEQ.ID.28) | | |
| 7 | 383-385 | RKS | AAA | +/-,- | +,- |
| 21 | 377, 381 | F(DL)F | Q(DL)H | + | + |
| | | (SEQ.ID.29) | (SEQ.ID.30) | | |
| 66 | 382 | I | A | + | + |
| 67 | 383 | R | A | + | +/- |
| 68 | 384 | K | A | + | + |
| 69 | 385 | S | A | | |
| 102 | 383, 384 | RK | DD | | |

| β-strand B | | | | | |
|---|---|---|---|---|---|
| 8 | 387, 389 | T(I)T | A(I)A | +/-,+ | - |
| 70 | 387 | T | A | + | +/-,+ |
| 71 | 389 | T | A | + | + |

| Loop BC | | | | | |
|---|---|---|---|---|---|
| 2 | 396-401 | APSKGT (SEQ.ID.31) | SHEDPQ (SEQ.ID.32) | | |

| β-strand C | | | | | |
|---|---|---|---|---|---|
| 9 | 403, 405 | N(L)T | A(L)A | + | + |

| Loop CD | | | | | |
|---|---|---|---|---|---|
| 3 | 407-420 | SRASGKPVNHS (SEQ.ID.33) | YVDGVQVHNAK (SEQ.ID.34) | +/- | - |
| 55 | 407-415 | SR(A)S(G)K (SEQ.ID.35) | AA(A)A(G)A (SEQ.ID.36) | +/- | + |
| 59 | 407 | S | A | + | + |
| 60 | 408 | R | A | + | - |
| 61 | 411 | S | A | + | + |
| 62 | 415 | K | A | + | - |
| 63 | 418 | N | A | +/- | + |
| 64 | 419 | H | A | + | + |
| 65 | 420 | S | A | +/- | + |
| 100 | 408 | R | E | | |
| 101 | 415 | K | D | | |

| β-strand D | | | | | |
|---|---|---|---|---|---|
| 6 | 423-428 | KEEKQR (SEQ.ID.37) | PREQQY (SEQ.ID.38) | + | + |
| 35 | 422 | R | A | + | + |
| 36 | 4423 | K | A | + | + |
| 37 | 424 | E | A | + | + |
| 38 | 425 | E | A | + | + |
| 39 | 426 | K | A | + | |
| 40 | 427 | Q | A | -,+/- | + |
| 41 | 428 | R | A | + | + |
| 75 | 423-425 | KEE | AAA | -,+/-,+ | + |
| 76 | 426-428 | KQR | AAA | | |
| 79 | 423,425,4 27 | KEEKQR (SEQ.ID.39) | AEAKAR (SEQ.ID.40) | | |
| 80 | | KEEKQR | KAEAQA | | |
| | 424,426,4 | (SEQ.ID.41) | (SEQ.ID.42) | | |
| 81 | 28 | K | P | | |
| 82 | | KEEKQR | AAEAQA | | |
| | 423, | (SEQ.ID.43) | (SEQ.ID.44) | | |
| | 423-427 | | | | |

| β-strand E | | | | | |
|---|---|---|---|---|---|
| 10 | 438,440 | T(S)T | A(S)A | + | + |

| Loop EF | | | | | |
|---|---|---|---|---|---|
| 4 | 444-453 | GTRDWIEGET (SEQ.ID.45) | LHQDWLDGKE (SEQ.ID.46) | - | - |
| 49 | 445 | T | A | + | + |
| 50 | 336 | R | A | + | - |
| 51 | 337 | D | A | + | +,- |
| | | | | +/- | |
| 52 | 450 | E | A | + | - |
| 53 | 452 | E | A | + | + |
| | | | | + | +/- |
| 77 | 445,446 | TR | AA | - | - |
| 78 | 450,452,453 | E(G)ET (SEQ.ID.47) | A(G)AA (SEQ.ID.48) | + + | + |
| 83 | | G | L | + | + |
| 84 | 444 | G | A | | |
| 85 | 444 | TRDWIEGET | HQDWLDGKE | - | + |
| | 445-453 | (SEQ.ID.49) | (SEQ.ID.50) | + | |
| 86 | | T | H | + | |
| 87 | 445 | TR | HQ | +/-,+ | |
| 88 | 445,446 | R | E | - | |
| 89 | 446 | E(G)ET | D(G)KE | +/-,- | +/- |
| | 450,452,4 | (SEQ.ID.51) | (SEQ.ID.52) | + | |
| 93 | 53 | D | R | +/-,- | |
| 94 | | E | R | | |
| 95 | 447 | E | R | | |
| 96 | 450 | T | R | | |
| 97 | 452 | D | N | | |
| 98 | 453 | E | Q | | |
| 99 | 447 | E | D | | |
| | 452 | | | | |
| | 452 | | | | |

| β-strand F | | | | | |
|---|---|---|---|---|---|
| 11 | 445,457,459 | Q(C)R(V)T (SEQ.ID.53) | A(C)A(V)A (SEQ.ID.54) | | |

| Loop FG | | | | | |
|---|---|---|---|---|---|
| 5 | 465-469 | RALM (SEQ.ID.55) | APIE (SEQ.ID.56) | | |

| β-strand G | | | | | |
|---|---|---|---|---|---|
| 12 | 471,473 | S(T)T | A(T)A | +,+ | |

| Fcε2 | | | | | |
|---|---|---|---|---|---|
| 13 | 329-331, 334-336 | QKH(WL)SDR (SEQ.ID.57) | AAA(WL)AAA (SEQ.ID.58) | +,+ | |

| Fcε4 | | | | | |
|---|---|---|---|---|---|
| 72 | 498-501 | PRAA (SEQ.ID.59) | QPRE (SEQ.ID.60) | | |
| 73 | 594-599 | ASPSQT (SEQ.ID.61) | LHNHY (SEQ.ID.62) | | |
| 74 | 595-599 | S(P)SQT (SEQ.ID.63) | A(P)AAA (SEQ.ID.64) | | |

| | | | | | |
|---|---|---|---|---|---|
| * Positive receptor binding indicated by "+", no binding by "-", and positive binding but less than unaltered is shown by "+/-". Where more than one assay was performed, results are separated by commas. | | | | | |

## Claims

1. An antibody which is capable of binding to FcεRII-bound IgE but is substantially incapable of binding to FcεRI-bound IgE, comprising at least a Fab region of a human recipient antibody into which have been substituted at one or more of positions 30, 30b, 30d, 33, 53, 91, 92, 93 and 94 in the light chain and positions 27, 28, 29, 29a, 31, 33, 34, 50, 52, 53, 54, 55, 58, 95, 97, 98, 99,100 and 101 in the heavy chain, residues from analogous positions in donor antibody MAE11, MAE13 or MAE15 having the light and heavy chain amino acid sequences as set out in SEQ ID NO:2 to 7, or a donor antibody having the characteristics possessed by the MAE11 antibody, in particular in binding soluble IgE, binding IgE-bearing B cells, blocking IgE binding to FcεRI and FcεRII, inhibiting in vitro IgE production and failing to bind to IgE coated basophils.

2. An antibody according to claim 1, wherein said donor antibody is MAE11.

3. An antibody according to claim 1 or claim 2, wherein the recipient human antibody has a Kabat human sub-group I kappa (light) chain and human sub-group III heavy chain.

4. An antibody according to any one of the preceding claims which is an IgG1, IgG2, IgG3 or IgG4 antibody.

5. An antibody according to claim 4 which is a complement-fixing IgG antibody, or an IgG antibody capable of participating in ADCC.

6. An antibody comprising the Fab heavy and light chain sequences of humae11ver.1, 2, 3, 4, 5, 6, 7, 7a, 8, 8a, 8b or 9, wherein humae11ver.1 has the heavy and light chain amino acid sequences as set out in SEQ ID NO: 8 and 9, and humaellver.2-9 have the heavy and light chain amino acid sequences of humae11ver.1, further incorporating the modifications shown in Table 5.

7. An antibody comprising Fab heavy and light chain amino acid sequences of humae11ver.1 as set out in SEQ ID NO: 8 and 9, said heavy chain sequence being substituted at position 60 with asparagine, at position 61 with proline and position 67 with isoleucine.

8. An antibody according to any one of claims 1 to 5, having additional substitution of one or more donor antibody framework residues at positions selected from 4, 13, 19, 33, 53 58, 78 and 104 in the light chain, and 24, 37, 50, 52, 58, 60, 61, 95, 78, 48, 49, 63, 67, 69, 82 and 82c in the heavy chain.

9. An antibody according to claim 8, having donor framework hydrophobic residues at positions 13, 19, 58, 78 and 104 in the light chain and 48, 49, 63, 67, 69, 78, 82 and 82c in the heavy chain.

10. An antibody according to claim 8, having donor residues at positions 4, 33 and 53 in the light chain and 24, 37, 50, 52, 58, 60, 61 and 95 in the heavy chain.

11. The antibody of any one of claims 1 to 5 and 8 to 10, having substitution, deletion or insertion of a donor antibody residue at or adjacent to sites 30, 30b, 30d, 33, 55, 57, 58, 78, 93, 94 or 104 in the light chain and/or sites 24, 37, 48, 49, 54, 57, 60, 61, 63, 65, 67, 69, 78, 82, 82c, 97, 100a or 100c in the heavy chain.

12. An antibody according to claim 11, wherein V_{H} 78 is substituted with phenylalanine.

13. An antibody according to claim 11, wherein V_{H} 78 is substituted with leucine, valine, isoleucine, methionine or alanine.

14. An antibody according to claim 11, wherein V_{H} 60 is substituted with asparagine.

15. An antibody according to claim 11, wherein V_{H} 60 is substituted with glutamine, histidine, lysine or arginine.

16. An antibody according to claim 11, wherein V_{H} 61 is substituted with proline.

17. An antibody according to claim 11, wherein V_{H} 61 is substituted with glycine, alanine, valine, leucine or isoleucine.

18. An antibody according to claim 8, wherein residues are imported from the donor MAE11, including four inserts in V_{L1} 30a-30d as well as V_{L3} 91-94, V_{H1} 27-29, 29a, 31, 33 and 34, V_{H2} 53-55 and V_{H3} 97-101.

19. An antibody according to claim 11, wherein any of V_{H} positions 97, 100a and 100c has a basic residue other than histidine, preferably lysine or arginine.

20. An antibody according to claim 11, wherein any of V_{H} positions 97, 100a and 100c has a residue selected from alanine, glycine, valine, isoleucine, serine, threonine, aspartic acid, glutamic acid, asparagine, glutamine, methionine, phenylalanine, tyrosine, tryptophan or proline.

21. An antibody according to claim 11, wherein any of V_{L} positions 30, 30b and 30d are glutamic acid.

22. An antibody according to claim 11, wherein any of V_{L} positions 30, 30b and 30d has a residue selected from alanine, glycine, valine, isoleucine, serine, threonine, asparagine, glutamine, methionine, phenylalanine, tyrosine, tryptophan or proline.

23. An antibody according to claim 11, wherein any of V_{L} positions 30, 30b and 30d are histidine and any of V_{H} positions 97, 100a and 100c are aspartic acid.

24. An antibody according to claim 11, wherein residues are inserted adjacent to any of V_{H} residues at positions 97, 100a, 100c, 60 or 61 or V_{L} residues at positions 30, 30b, 30d or 78.

25. An antibody according to claim 24, wherein the inserted residues are of like kind, that is acid residues inserted adjacent to V_{L} 30, 30b or 30d or basic residues adjacent to V_{H} 97, 100a or 100c.

26. An antibody according to claim 11, wherein residues at any of V_{L} 30, 30b or 30d or 78 or of V_{H} 97, 100a, 100c, 60 or 61 are deleted.

27. An antibody according to any one of the preceding claims, which is a bispecific antibody.

28. An antibody according to any one of the preceding claims, which is monovalent for FcεRII-bound IgE, and is capable of an immunoglobulin effector function and comprises an Fc domain containing at least two heavy chains.

29. An antibody according to any one of the preceding claims, comprising a human consensus heavy chain and light chain sequence.

30. An antibody according to any one of the preceding claims, which has an IgE affinity which is substantially the same as or greater than that of MAE11 for IgE.

31. A method of making an antibody which is capable of binding to FcεRII-bound IgE but is substantially incapable of binding to FcεRI-bound IgE, comprising substituting into at least a Fab region of a human recipient antibody, at one or more of positions 30, 30b, 30d, 33, 53, 91, 92, 93 and 94 in the light chain and positions 27, 28, 29, 29a, 31, 33, 34, 50, 52, 53, 54, 55, 58, 95, 97, 98, 99, 100 and 101 in the heavy chain, residues from analogous positions in donor antibody MAE11, MAE13 or MAE15 having the light and heavy chain amino acid sequences as set out in SEQ ID NO:2 to 7, or a donor antibody having the characteristics possessed by the MAE11 antibody, in particular in binding soluble IgE, binding IgE-bearing B cells, blocking IgE binding to FcεRI and FcεRII, inhibiting in vitro IgE production and failing to bind to IgE coated basophils.

32. A method according to claim 31, wherein said donor antibody is MAE11.

33. A method according to claim 31 or claim 32, wherein the recipient human antibody has a Kabat human sub-group I kappa (light) chain and human sub-group III heavy chain.

34. A method according to any one of claims 31 to 33 wherein the antibody thus produced is an IgG1, IgG2, IgG3 or IgG4 antibody.

35. A method according to claim 34 wherein the antibody thus produced is a complement-fixing IgG antibody, or an IgG antibody capable of participating in ADCC.

36. A method according to any one of claims 31 to 35, comprising additional substitution of one or more donor antibody framework residues at positions selected from 4, 13, 19, 33, 53 58, 78 and 104 in the light chain, and 24, 37, 50, 52, 58, 60, 61, 95, 78, 48, 49, 63, 67, 69, 82 and 82c in the heavy chain.

37. A method according to claim 36, wherein the antibody thus produced has donor framework hydrophobic residues at positions 13, 19, 58, 78 and 104 in the light chain and 48, 49, 63, 67, 69, 78, 82 and 82c in the heavy chain.

38. A method according to claim 36, wherein the antibody thus produced has donor residues at positions 4, 33 and 53 in the light chain and 24, 37, 50, 52, 58, 60, 61 and 95 in the heavy chain.

39. A method according to any one of claims 31 to 38, comprising substitution, deletion or insertion of a donor antibody residue at or adjacent to sites 30, 30b, 30d, 33, 55, 57, 58, 78, 93, 94 or 104 in the light chain and/or sites 24, 37, 48, 49, 54, 57, 60, 61, 63, 65, 67, 69, 78, 82, 82c, 97, 100a or 100c in the heavy chain.

40. A method according to claim 39, wherein V_{H} 78 is substituted with phenylalanine.

41. A method according to claim 39, wherein V_{H} 78 is substituted with leucine, valine, isoleucine, methionine or alanine.

42. A method according to claim 39, wherein V_{H} 60 is substituted with asparagine.

43. A method according to claim 39, wherein V_{H} 60 is substituted with glutamine, histidine, lysine or arginine.

44. A method according to claim 39, wherein V_{H} 61 is substituted with proline.

45. A method according to claim 39, wherein V_{H} 61 is substituted with glycine, alanine, valine, leucine or isoleucine.

46. A method according to claim 36, wherein residues are imported from the donor MAE11, including four inserts in V_{L1} 30a-30d as well as 91-94, V_{H1} 27-29, 29a, 31, 33 and 34, V_{H2} 53-55 and V_{H3} 97-101.

47. A method according to claim 39, wherein in the antibody thus produced, any of V_{H} positions 97, 100a and 100c has a basic residue other than histidine, preferably lysine or arginine.

48. A method according to claim 39, wherein in the antibody thus produced, any of V_{H} positions 97, 100a and 100c has a residue selected from alanine, glycine, valine, isoleucine, serine, threonine, aspartic acid, glutamic acid, asparagine, glutamine, methionine, phenylalanine, tyrosine, tryptophan or proline.

49. A method according to claim 39, wherein in the antibody thus produced, any of V_{L} positions 30, 30b and 30d are glutamic acid.

50. A method according to claim 39, wherein in the antibody thus produced, any of V_{L} positions 30, 30b and 30d has a residue selected from alanine, glycine, valine, isoleucine, serine, threonine, asparagine, glutamine, methionine, phenylalanine, tyrosine, tryptophan or proline.

51. A method according to claim 39, wherein in the antibody thus produced, any of V_{L}, positions 30, 30b and 30d are histidine and any of V_{H} positions 97, 100a and 100c are aspartic acid.

52. A method according to claim 39, wherein residues are inserted adjacent to any of V_{H} residues at positions 97, 100a, 100c, 60 or 61 or V_{L} residues at positions 30, 30b, 30d or 78.

53. A method according to claim 52, wherein the inserted residues are of like kind, that is acid residues inserted adjacent to V_{L} 30, 30b or 30d or basic residues adjacent to V_{H} 97, 100a or 100c.

54. A method according to claim 39, wherein residues at any of V_{L} 30, 30b or 30d or 78 or of V_{H} 97, 100a, 100c, 60 or 61 are deleted.

55. A method according to any one of claims 31 to 54, wherein the antibody thus produced is a bispecific antibody.

56. A method according to any one of claims 31 to 55, wherein the antibody thus produced is monovalent for FcεRII-bound IgE, and is capable of an immunoglobulin effector function and comprises an Fc domain containing at least two heavy chains.

57. A method according to any one of claims 31 to 56, wherein the antibody thus produced comprises a human consensus heavy chain and light chain sequence.

58. A method according to any one of claims 31 to 57, wherein the antibody thus produced has an IgE affinity which is substantially the same as or greater than that of MAE11 for IgE.

## Patentansprüche

1. Antikörper, der zur Bindung an FcεRII-gebundenen IgE fähig ist, aber im Wesentlichen unfähig zur Bindung an FcεRl-gebundenen IgE ist, umfassend zumindest eine Fab-Region eines Human-Rezipienten-Antikörpers, worin an einer oder mehreren der Positionen 30, 30b, 30d, 33, 53, 91, 92, 93 und 94 in der Leichtkette und der Positionen 27, 28, 29, 29a, 31, 33, 34, 50, 52, 53, 54, 55, 58, 95, 97, 98, 99, 100 und 101 in der Schwerkette Reste von analogen Positionen im Donor-Antikörper MAE11, MAE13 oder MAE15 substituiert worden sind, die Leicht- und Schwerketten-Aminosäuresequenzen wie in Seq.-ID Nr. 2 bis 7 dargelegt aufweisen, oder in einem Donor-Antikörper, der jene Eigenschaften aufweist, die der MAE11-Antikörper besitzt, insbesondere was die Bindung von löslichem IgE, Bindung von IgE-tragenden B-Zellen, Blockierung der IgE-Bindung an FcεRI und FcεRII, Hemmung der In-vitro-lgE-Produktion und Nichtbindung an IgE-beschichtete Basophile betrifft.

2. Antikörper nach Anspruch 1, worin der Donor-Antikörper MAE11 ist.

3. Antikörper nach Anspruch 1 oder 2, worin der Rezipienten-Human-Antikörper eine Kabat-Human-Untergruppen I-κ-(Leicht-)Kette und Human-Untergruppen lll-Schwerkette aufweist.

4. Antikörper nach einem der vorangegangenen Ansprüche, der ein IgG1-, IgG2-, IgG3- oder IgG4-Antikörper ist.

5. Antikörper nach Anspruch 4, der ein Komplement-fixierender IgG-Antikörper oder ein zur Teilnahme an ADCC fähiger IgG-Antikörper ist.

6. Antikörper, der die Fab-Schwer- und -Leichtkettensequenzen von humae1 1ver.1, 2, 3, 4, 5, 6, 7, 7a, 8, 8a, 8b oder 9 umfasst, worin humae11ver.1 die Schwer- und Leichtketten-Aminosäuresequenzen wie in Seq.-ID Nr. 8 und 9 dargelegt aufweist und humae11ver.2 bis 9 die Schwer- und Leichtketten-Aminosäuresequenzen von humae11-ver.1 mit den in Tabelle 5 angeführten Modifikationen aufweisen.

7. Antikörper, der Fab-Schwer- und -Leichtketten-Aminosäuresequenzen von humae11ver.1 wie in Seq.-ID Nr. 8 und 9 dargelegt umfasst, wobei die Schwerketten-Sequenz an Position 60 mit Asparagin, an Position 61 mit Prolin und an Position 67 mit Isoleucin substituiert ist.

8. Antikörper nach einem der Ansprüche 1 bis 5, der zusätzliche Substitution eines oder mehrerer Donor-Antikörper-Rahmenreste an Positionen aufweist, die aus 4, 13, 19, 33, 53, 58, 78 und 104 in der Leichtkette und 24, 37, 50, 52, 58, 60, 61, 95, 78, 48, 49, 63, 67, 69, 82 und 82c in der Schwerkette ausgewählt sind.

9. Antikörper nach Anspruch 8, der hydrophobe Donor-Rahmenreste an den Positionen 13, 19, 58, 78 und 104 in der Leichtkette und 48, 49, 63, 67, 69, 78, 82 und 82c in der Schwerkette aufweist.

10. Antikörper nach Anspruch 8, der Donorreste an den Positionen 4, 33 und 53 in der Leichtkette und 24, 37, 50, 52, 58, 60, 61 und 95 in der Schwerkette aufweist.

11. Antikörper nach einem der Ansprüche 1 bis 5 und 8 bis 10, der Substitution, Deletion oder Insertion eines Donor-Antikörper-Rests an oder benachbart zu den Orten 30, 30b, 30d, 33, 55, 57, 58, 78, 93, 94 oder 104 in der Leichtkette und/oder den Orten 24, 37, 48, 49, 54, 57, 60, 61, 63, 65, 67, 69, 78, 82, 82c, 97, 100a oder 100c in der Schwerkette aufweist.

12. Antikörper nach Anspruch 11, worin V_{H} 78 mit Phenylalanin substituiert ist.

13. Antikörper nach Anspruch 11, worin V_{H} 78 mit Leucin, Valin, Isoleucin, Methionin oder Alanin substituiert ist.

14. Antikörper nach Anspruch 11, worin V_{H} 60 mit Asparagin substituiert ist.

15. Antikörper nach Anspruch 11, worin V_{H} 60 mit Glutamin, Histidin, Lysin oder Arginin substituiert ist.

16. Antikörper nach Anspruch 11, worin V_{H} 61 mit Prolin substituiert ist.

17. Antikörper nach Anspruch 11, worin V_{H} 61 mit Glycin, Alanin, Valin, Leucin oder Isoleucin substituiert ist.

18. Antikörper nach Anspruch 8, worin Reste vom Donor MAE11 importiert sind, einschließlich vier Inserts in V_{ω} 30a-30d sowie V_{ω} 91-94, V_{H1}27-29, 29a, 31, 33 und 34, V_{H2} 53-55 und V_{H3} 97-101.

19. Antikörper nach Anspruch 11, worin eine beliebige der V_{H}-Positionen 97, 100a und 100c einen anderen basischen Rest als Histidin, vorzugsweise Lysin oder Arginin, aufweist.

20. Antikörper nach Anspruch 11, worin eine beliebige der V_{H}-Positionen 97, 100a und 100c einen Rest aufweist, der aus Alanin, Glycin, Valin, Isoleucin, Serin, Threonin, Asparaginsäure, Glutaminsäure, Asparagin, Glutamin, Methionin, Phenylalanin, Tyrosin Tryptophan und Prolin ausgewählt ist.

21. Antikörper nach Anspruch 11, worin beliebige der V_{L}-Positionen 30, 30b und 30d Glutaminsäure sind.

22. Antikörper nach Anspruch 11, worin eine beliebige der V_{L}-Positionen 30, 30b und 30d einen Rest aufweist, der aus Alanin, Glycin, Valin, Isoleucin, Serin, Threonin, Asparagin, Glutamin, Methionin, Phenylalanin, Tyrosin, Tryptophan und Prolin ausgewählt ist.

23. Antikörper nach Anspruch 11, worin beliebige der V_{L}-Positionen 30, 30b und 30d Histidin und beliebige der V_{H}-Positionen 97, 100a und 100c Asparaginsäure sind.

24. Antikörper nach Anspruch 11, worin Reste benachbart zu beliebigen der V_{H}-Reste an den Positionen 97, 100a, 100c, 60 oder 61 oder der V_{L}-Reste an den Positionen 30, 30b, 30d oder 78 insertiert sind.

25. Antikörper nach Anspruch 24, worin die insertierten Reste gleichartig sind, d.h. saure Reste benachbart zu V_{L} 30, 30b oder 30d oder basische Reste benachbart zu V_{H} 97, 100a oder 100c insertiert sind.

26. Antikörper nach Anspruch 11, worin Reste an beliebigen der Positionen V_{L} 30, 30b oder 30d oder 78 oder V_{H} 97, 100a, 100c, 60 oder 61 deletiert sind.

27. Antikörper nach einem der vorangegangenen Ansprüche, der ein bispezifischer Antikörper ist.

28. Antikörper nach einem der vorangegangenen Ansprüche, der für FcεRII-gebundenen IgE einwertig ist, zu einer lmmunglobulin-Effektorfunktion fähig ist und eine Fc-Domäne umfasst, die zumindest zwei Schwerketten enthält.

29. Antikörper nach einem der vorangegangenen Ansprüche, der eine Human-Konsens-Schwerketten- und -Leichtkettensequenz umfasst.

30. Antikörper nach einem der vorangegangenen Ansprüche, der eine IgE-Affinität aufweist, die im Wesentlichen gleich stark wie oder stärker als jene von MAE11 für IgE ist.

31. Verfahren zur Herstellung eines Antikörpers, der zur Bindung an FcεRll-gebundenen IgE fähig ist, aber im Wesentlichen zur Bindung an FcεRI-gebundenen IgE unfähig ist, umfassend das Substituieren in zumindest eine Fab-Region eines Human-Rezipienten-Antikörpers an einer oder mehreren der Positionen 30, 30b, 30d, 33, 53, 91, 92, 93 und 94 in der Leichtkette und der Positionen 27, 28, 29, 29a, 31, 33, 34, 50, 52, 53, 54, 55, 58, 95, 97, 98, 99, 100 und 101 in der Schwerkette, von Resten von analogen Positionen im Donor-Antikörper MAE11, MAE13 oder MAE15 mit den Leicht- und Schwerketten-Aminosäuresequenzen wie in Seq.-ID Nr. 2 bis 7 dargelegt oder in einem Donor-Antikörper, der jene Eigenschaften aufweist, die der MAE11-Antikörper besitzt, insbesondere was die Bindung von löslichem IgE, Bindung von IgE-tragenden B-Zellen, Blockierung der IgE-Bindung an FcεRI und FcεRII, Hemmung der In-vitro-lgE-Produktion und Nichtbindung an IgE-beschichtete Basophile betrifft.

32. Verfahren nach Anspruch 31, worin der Donor-Antikörper MAE11 ist.

33. Verfahren nach Anspruch 31 oder 32, worin der Rezipienten-Human-Antikörper eine Kabat-Human-Untergruppen l-κ-(Leicht-)Kette und eine Human-Untergruppen III-Schwerkette aufweist.

34. Verfahren nach einem der Ansprüche 31 bis 33, worin der so hergestellte Antikörper ein IgG1-, IgG2-, IgG3- oder IgG4-Antikörper ist.

35. Verfahren nach Anspruch 34, worin der so hergestellte Antikörper ein Komplement-fixierender IgG-Antikörper oder ein IgG-Antikörper ist, der zur Teilnahme an ADCC fähig ist.

36. Verfahren nach einem der Ansprüche 31 bis 35, umfassend zusätzliche Substitution eines oder mehrerer Donor-Antikörper-Rahmenreste an Positionen, die aus 4, 13, 19, 33, 53, 58, 78 und 104 in der Leichtkette und 24, 37, 50, 52, 58, 60, 61, 95, 78, 48, 49, 63, 67, 69, 82 und 82c in der Schwerkette ausgewählt sind.

37. Verfahren nach Anspruch 36, worin der so hergestellte Antikörper hydrophobe Donor-Rahmenreste an den Positionen 13, 19, 58, 78 und 104 in der Leichtkette und 48, 49, 63, 67, 69, 78, 82 und 82c in der Schwerkette aufweist.

38. Verfahren nach Anspruch 36, worin der so hergestellte Antikörper Donor-Reste an den Positionen 4, 33, und 53 in der Leichtkette und 24, 37, 50, 52, 58, 60, 61 und 95 in der Schwerkette aufweist.

39. Verfahren nach einem der Ansprüche 31 bis 38, umfassend die Substitution, Deletion oder Insertion eines Donor-Antikörper-Rests an oder benachbart zu den Orten 30, 30b, 30d, 33, 55, 57, 58, 78, 93, 94 oder 104 in der Leichtkette und/oder den Orten 24, 37, 48, 49, 54, 57, 60, 61, 63, 65, 67, 69, 78, 82, 82c, 97, 100a oder 100c in der Schwerkette.

40. Verfahren nach Anspruch 39, worin V_{H} 78 mit Phenylalanin substituiert wird.

41. Verfahren nach Anspruch 39, worin V_{H} 78 mit Leucin, Valin, Isoleucin, Methionin oder Alanin substituiert wird.

42. Verfahren nach Anspruch 39, worin V_{H} 60 mit Asparagin substituiert wird.

43. Verfahren nach Anspruch 39, worin V_{H} 60 mit Glutamin, Histidin, Lysin oder Arginin substituiert wird.

44. Verfahren nach Anspruch 39, worin V_{H} 61 mit Prolin substituiert wird.

45. Verfahren nach Anspruch 39, worin V_{H} 61 mit Glycin, Alanin, Valin, Leucin oder Isoleucin substituiert wird.

46. Verfahren nach Anspruch 36, worin Reste vom Donor MAE11 importiert werden, einschließlich vier Inserts in V_{L1} 30a-30d sowie 91-94, V_{H1} 27-29, 29a, 31, 33 und 34, V_{H2} 53-55 und V_{H3} 97-101.

47. Verfahren nach Anspruch 39, worin im so hergestellten Antikörper eine beliebige der V_{H}-Positionen 97, 100a und 100c einen anderen basischen Rest als Histidin, vorzugsweise Lysin oder Arginin, aufweist.

48. Verfahren nach Anspruch 39, worin im so hergestellten Antikörper eine beliebige der V_{H}-Positionen 97, 100a und 100c einen Rest aufweist, der aus Alanin, Glycin, Valin, Isoleucin, Serin, Threonin, Asparaginsäure, Glutaminsäure, Asparagin, Glutamin, Methionin, Phenylalanin, Tyrosin, Tryptophan und Prolin ausgewählt ist.

49. Verfahren nach Anspruch 39, worin im so hergestellten Antikörper beliebige der V_{L}-Positionen 30, 30b und 30d Glutaminsäure sind.

50. Verfahren nach Anspruch 39, worin im so hergestellten Antikörper eine beliebige der V_{L}-Positionen 30, 30b und 30d einen Rest aufweist, der aus Alanin, Glycin, Valin, Isoleucin, Serin, Threonin, Asparagin, Glutamin, Methionin, Phenylalanin, Tyrosin, Tryptophan und Prolin ausgewählt ist.

51. Verfahren nach Anspruch 39, worin im so hergestellten Antikörper beliebige der V_{L}-Positionen 30, 30b und 30d Histidin und beliebige der V_{H}-Positionen 97, 100a und 100c Asparaginsäure sind.

52. Verfahren nach Anspruch 39, worin Reste benachbart zu beliebigen der V_{H}-Reste an den Positionen 97, 100a, 100c, 60 oder 61 oder der V_{L}-Reste an den Positionen 30, 30b, 30d oder 78 insertiert werden.

53. Verfahren nach Anspruch 52, worin die insertierten Reste gleichartig sind, d.h. saure Reste benachbart zu V_{L} 30, 30b oder 30d oder basische Reste benachbart zu V_{H} 97, 100a oder 100c insertiert werden.

54. Verfahren nach Anspruch 39, worin Reste an beliebigen der Positionen V_{L} 30, 30b oder 30d oder 78 oder V_{H} 97, 100a, 100c, 60 oder 61 deletiert werden.

55. Verfahren nach einem der Ansprüche 31 bis 54, worin der so hergestellte Antikörper ein bispezifischer Antikörper ist.

56. Verfahren nach einem der Ansprüche 31 bis 55, worin der so hergestellte Antikörper für FcεRII-gebundenen IgE einwertig ist, zu einer Immunglobulin-Effektorfunktion fähig ist und eine Fc-Domäne umfasst, die zumindest zwei Schwerketten enthält.

57. Verfahren nach einem der Ansprüche 31 bis 56, worin der so hergestellte Antikörper eine Human-Konsens-Schwerketten- und -Leichtkettensequenz umfasst.

58. Verfahren nach einem der Ansprüche 31 bis 57, worin der so hergestellte Antikörper eine IgE-Affinität aufweist, die im Wesentlichen gleich stark wie oder stärker als jene von MAE11 für IgE ist.

## Revendications

1. Anticorps qui est capable de se lier à une IgE liée à FcεRII, mais qui est pratiquement incapable de se lier à une IgE liée à FcεRI, comprenant au moins une région Fab d'un anticorps de receveur humain auquel ont été introduits par substitution au niveau d'une ou plusieurs des positions 30, 30b, 30d, 33, 53, 91, 92, 93 et 94 dans la chaîne légère et des positions 27, 28, 29, 29A, 31, 33, 34, 50, 52, 53, 54, 55, 58, 95, 97, 98, 99, 100 et 101 dans la chaîne lourde des résidus provenant de positions analogues dans l'anticorps donneur MAE11, MAE13 ou MAE15 ayant les séquences d'amino-acides de chaînes légère et lourde représentées dans les SEQ ID Nos 2 à 7, ou un anticorps donneur ayant les caractéristiques présentées par l'anticorps MAE11, en particulier dans la liaison d'une IgE soluble, la liaison de lymphocytes B porteurs de IgE, le blocage de la liaison d'une IgE à FcεRI et FcεRII, l'inhibition in vitro de la production de IgE et l'incapacité de liaison à des cellules basophiles revêtues de IgE.

2. Anticorps suivant la revendication 1, dans lequel ledit anticorps donneur est l'anticorps MAE11.

3. Anticorps suivant la revendication 1 ou la revendication 2, dans lequel l'anticorps humain receveur possède une chaîne kappa (légère) de sous-groupe I humain et une chaîne lourde de sous-groupe III humain Kabat.

4. Anticorps suivant l'une quelconque des revendications précédentes, qui est un anticorps IgG1, IgG2, IgG3 ou IgG4.

5. Anticorps suivant la revendication 4, qui est un anticorps IgG de fixation du complément, ou un anticorps IgG capable de participer à une ADCC.

6. Anticorps comprenant les séquences de chaînes lourde et légère Fab de humae11ver. 1, 2, 3, 4, 5, 6, 7, 7a, 8, 8a, 8b ou 9, dans lequel humae11ver.1 possède les séquences d'amino-acides de chaînes lourde et légère représentées dans les SEQ ID Nos 8 et 9, et humae11ver. 2-9 possède les séquences d'amino-acides de chaînes lourde et légère de humae11ver.1, renfermant en outre les modifications indiquées sur le tableau 5.

7. Anticorps comprenant les séquences d'amino-acides de chaînes lourde et légère Fab de humae11ver.1 indiquées dans les SEQ ID Nos 8 et 9, ladite séquence de chaîne lourde étant substituée en position 60 avec l'asparagine, en position 61 avec la proline et en position 67 avec l'isoleucine.

8. Anticorps suivant l'une quelconque des revendications 1 à 5, présentant une substitution supplémentaire d'un ou plusieurs résidus d'ossature d'anticorps donneurs à des positions choisies entre les positions 4, 13, 19, 33, 53, 58, 78 et 104 dans la chaîne légère, et 24, 37, 50, 52, 58, 60, 61, 95, 78, 48, 49, 63, 67, 69, 82 et 82c dans la chaîne lourde.

9. Anticorps suivant la revendication 8, ayant des résidus hydrophobes d'ossature donneurs en positions 13, 19, 58, 78 et 104 dans la chaîne légère et 48, 49, 63, 67, 69, 78, 82 et 82c dans la chaîne lourde.

10. Anticorps suivant la revendication 8, ayant des résidus donneurs en positions 4, 33 et 53 dans la chaîne légère et 24, 37, 50, 52, 58, 60, 61 et 95 dans la chaîne lourde.

11. Anticorps suivant l'une quelconque des revendications 1 à 5 et 8 à 10, ayant une substitution, délétion ou insertion d'un résidu d'anticorps donneur au niveau du, ou en position adjacente au, site 30, 30b, 30d, 33, 55, 57, 58, 78, 93, 94 ou 104 dans la chaîne légère et/ou site 24, 37, 48, 49, 54, 57, 60, 61, 63, 65, 67, 69, 78, 82, 82c, 97, 100a ou 100c dans la chaîne lourde.

12. Anticorps suivant la revendication 11, dans lequel V_{H} 78 est substitué avec la phénylalanine.

13. Anticorps suivant la revendication 11, dans lequel V_{H} 78 est substitué avec la leucine, la valine, l'isoleucine, la méthionine ou l'alanine.

14. Anticorps suivant la revendication 11, dans lequel V_{H} 60 est substitué avec l'asparagine.

15. Anticorps suivant la revendication 11, dans lequel V_{H} 60 est substitué avec la glutamine, l'histidine, la lysine ou l'arginine.

16. Anticorps suivant la revendication 11, dans lequel V_{H} 61 est substitué avec la proline.

17. Anticorps suivant la revendication 11, dans lequel V_{H} 61 est substitué avec la glycine, l'alanine, la valine, la leucine ou l'isoleucine.

18. Anticorps suivant la revendication 8, dans lequel les résidus sont importés à partir du donneur MAE11, comprenant quatre segments d'insertion dans V_{L1} 30a-30d ainsi que V_{L3} 91-94, V_{H1} 27-29, 29a, 31, 33 et 34, V_{H2} 53-55 et V_{H3} 97-101.

19. Anticorps suivant la revendication 11, dans lequel n'importe lesquelles des positions V_{H} 97, 100a et 100c possèdent un résidu basique autre que l'histidine, de préférence la lysine ou l'arginine.

20. Anticorps suivant la revendication 11, dans lequel n'importe lesquelles des positions V_{H} 97, 100a et 100c possèdent un résidu choisi entre l'alanine, la glycine, la valine, l'isoleucine, la sérine, la thréonine, l'acide aspartique, l'acide glutamique, l'asparagine, la glutamine, la méthionine, la phénylalanine, la tyrosine, le tryptophane et la proline.

21. Anticorps suivant la revendication 11, dans lequel n'importe lesquelles des positions V_{L} 30, 30b et 30d sont occupées par l'acide glutamique.

22. Anticorps suivant la revendication 11, dans lequel n'importe lesquelles des positions V_{L} 30, 30b et 30d possèdent un résidu choisi entre l'alanine, la glycine, la valine, l'isoleucine, la sérine, la thréonine, l'asparagine, la glutamine, la méthionine, la phénylalanine, la tyrosine, le tryptophane et la proline.

23. Anticorps suivant la revendication 11, dans lequel n'importe lesquelles des positions V_{L} 30, 30b et 30d sont occupées par l'histidine et n'importe lesquelles des positions V_{H} 97, 100a et 100c sont occupées par l'acide aspartique.

24. Anticorps suivant la revendication 11, dans lequel les résidus sont insérés en position adjacente à n'importe lesquels des résidus V_{H} aux positions 97, 100a, 60 et 61 ou résidus V_{L} en positions 30, 30b, 30d et 78.

25. Anticorps suivant la revendication 24, dans lequel les résidus insérés sont de même type, c'est-à-dire des résidus acides insérés en position adjacente à V_{L} 30, 30b ou 30d ou des résidus basiques adjacents à V_{H} 97, 100a ou 100c.

26. Anticorps suivant la revendication 11, dans lequel des résidus à n'importe lesquelles des positions V_{L} 30, 30b ou 30d ou 78 ou des positions V_{H} 97, 100a, 100c, 60 ou 61 sont éliminés par délétion.

27. Anticorps suivant l'une quelconque des revendications précédentes, qui est un anticorps bispécifique.

28. Anticorps suivant l'une quelconque des revendications précédentes, qui est monovalent pour une IgE liée à FcεRII, et qui est capable de présenter une fonction effectrice d'immunoglobuline et qui comprend un domaine Fc contenant au moins deux chaînes lourdes.

29. Anticorps suivant l'une quelconque des revendications précédentes, comprenant une séquence de chaînes lourdes et chaînes légères consensus humaine.

30. Anticorps suivant l'une quelconque des revendications précédentes, qui présente une affinité pour IgE qui est pratiquement identique ou supérieure à celle de MAE11 pour une IgE.

31. Procédé pour la préparation d'un anticorps qui est capable de se lier à une IgE liée FcεRII, mais qui est pratiquement incapable de se lier à une IgE liée à FcεRI, comprenant la substitution dans au moins une région Fab d'un anticorps receveur humain, au niveau d'une ou plusieurs des positions 30, 30b, 30d, 33, 53, 91, 92, 93 et 94 dans la chaîne légère et des positions 27, 28, 29, 29a, 31, 33, 34, 50, 52, 53, 54, 55, 58, 95, 97, 98, 99, 100 et 101 dans la chaîne lourde, des résidus provenant de positions analogues dans un anticorps donneur MAE11, MAE13 ou MAE15 ayant les séquences d'amino-acides de chaînes légère et lourde représentées dans les SEQ ID Nos 2 à 7, ou un anticorps donneur ayant les caractéristiques présentées par l'anticorps MAE11, en particulier dans la liaison d'une IgE soluble, la liaison de lymphocytes B porteurs de IgE, le blocage de la liaison d'une IgE à FcεRI et FcεRII, l'inhibition in vitro de la production de IgE et l'incapacité à se lier à des cellules basophiles revêtues de IgE.

32. Procédé suivant la revendication 31, dans lequel l'anticorps donneur est un anticorps MAE11.

33. Procédé suivant la revendication 31 ou la revendication 32, dans lequel l'anticorps humain receveur possède une chaîne kappa (légère) de sous-groupe I humain et une chaîne lourde de sous-groupe III humain Kabat.

34. Procédé suivant l'une quelconque des revendications 31 à 33, dans lequel l'anticorps ainsi poduit est un anticorps IgG1, IgG2, IgG3 ou IgG4.

35. Procédé suivant la revendication 34, dans lequel l'anticorps ainsi produit est un anticorps IgG de fixation du complément, ou un anticorps IgG capable de participer à une ADCC.

36. Procédé suivant l'une quelconque des revendications 31 à 35, comprenant une substitution supplémentaire au niveau d'un ou plusieurs résidus d'ossature d'anticorps donneurs à des positions choisies entre les positions 4, 13, 19, 33, 53, 58, 78 et 104 dans la chaîne légère, et les positions 24, 37, 50, 52, 58, 60, 61, 95, 78, 48, 49, 63, 67, 69, 82 et 82c dans la chaîne lourde.

37. Procédé suivant la revendication 36, dans lequel l'anticorps ainsi produit possède des résidus hydrophobes d'ossature donneurs en positions 13, 19, 58, 78 et 104 dans la chaîne légère et 48, 49, 63, 67, 69, 78, 82 et 82c dans la chaîne lourde.

38. Procédé suivant la revendication 36, dans lequel l'anticorps ainsi produit possède des résidus donneurs en positions 4, 33 et 53 dans la chaîne légère et 24, 37, 50, 52, 58, 60, 61 et 95 dans la chaîne lourde.

39. Procédé suivant l'une quelconque des revendications 31 à 38, comprenant la substitution, la délétion ou l'insertion d'un résidu d'anticorps donneur au niveau des, ou en position adjacente aux, sites 30, 30b, 30d, 33, 55, 57, 58, 78, 93, 94 ou 104 dans la chaîne légère et/ou sites 24, 37, 48, 49, 54, 57, 60, 61, 63, 65, 67, 69, 78, 82, 82c, 97, 100a ou 100c dans la chaîne lourde.

40. Procédé suivant la revendication 39, dans lequel V_{H} 78 est substitué avec la phénylalanine.

41. Procédé suivant la revendication 39, dans lequel V_{H} 78 est substitué avec la leucine, la valine, l'isoleucine, la méthionine ou l'alanine.

42. Procédé suivant la revendication 39, dans lequel V_{H} 60 est substitué avec l'asparagine.

43. Procédé suivant la revendication 39, dans lequel V_{H} 60 est substitué avec la glutamine, l'histidine, la lysine ou l'arginine.

44. Procédé suivant la revendication 39, dans lequel V_{H} 61 est substitué avec la proline.

45. Procédé suivant la revendication 39, dans lequel V_{H} 61 est substitué avec la glycine, l'alanine, la valine, la leucine ou l'isoleucine.

46. Procédé suivant la revendication 36, dans lequel les résidus sont importés à partir du donneur MAE11, comprenant quatre segments d'insertion dans V_{L1} 30a-30d ainsi que 91-94, V_{H1} 27-29, 29a, 31, 33 et 34, V_{H2} 53-55 et V_{H2} 97-101.

47. Procédé suivant la revendication 39, dans lequel, dans l'anticorps ainsi produit, n'importe lesquelles des positions V_{H} 97, 100a et 100c possèdent un résidu basique autre que l'histidine, de préférence la lysine ou l'arginine.

48. Procédé suivant la revendication 39, dans lequel, dans l'anticorps ainsi produit, n'importe lesquelles des positions V_{H} 97, 100a et 100c possèdent un résidu choisi entre l'alanine, la glycine, la valine, l'isoleucine, la sérine, la thréonine, l'acide aspartique, l'acide glutamique, l'asparagine, la glutamine, la méthionine, la phénylalanine, la tyrosine, le tryptophane ou la proline.

49. Procédé suivant la revendication 39, dans lequel, dans l'anticorps ainsi produit, n'importe lesquelles des positions V_{L} 30, 30b et 30d sont occupées par l'acide glutamique.

50. Procédé suivant la revendication 39, dans lequel, dans l'anticorps ainsi produit, n'importe lesquelles des positions V_{L} 30, 30b et 30d possèdent un résidu choisi entre l'alanine, la glycine, la valine, l'isoleucine, la sérine, la thréonine, l'asparagine, la glutamine, la méthionine, la phénylalanine, la tyrosine, le tryptophane ou la proline.

51. Procédé suivant la revendication 39, dans lequel, dans l'anticorps ainsi produit, n'importe lesquelles des positions V_{L} 30, 30b et 30d sont occupées par l'histidine et n'importe lesquelles des positions V_{H} 97, 100a et 100c sont occupées par l'acide aspartique.

52. Procédé suivant la revendication 39, dans lequel des résidus sont insérés en position adjacente à n'importe lesquels des résidus V_{H} en position 97, 100a, 60 ou 61 ou résidus V_{L} en position 30, 30b, 30d ou 78.

53. Procédé suivant la revendication 52, dans lequel les résidus insérés sont de même type, c'est-à-dire des résidus acides insérés en position adjacente à V_{L} 30, 30b ou 30d ou des résidus basiques insérés en position adjacente à V_{H} 97, 100a ou 100c.

54. Procédé suivant la revendication 39, dans lequel des résidus au niveau de n'importe lesquelles des positions V_{L} 30, 30b, 30d ou 78 ou des positions V_{H} 97, 100a, 100c, 60 et 61 sont éliminés par délétion.

55. Procédé suivant l'une quelconque des revendications 31 à 54, dans lequel l'anticorps ainsi produit est un anticorps bispécifique.

56. Procédé suivant l'une quelconque des revendications 31 à 55, dans lequel l'anticorps ainsi produit est monovalent pour une IgE liée à FcεRII, et est capable de présenter une fonction effectrice d'immunoglobuline et comprend un domaine Fc contenant au moins deux chaînes lourdes.

57. Procédé suivant l'une quelconque des revendications 31 à 56, dans lequel l'anticorps ainsi produit comprend une séquence de chaînes lourdes et chaînes légères consensus humaine.

58. Procédé suivant l'une quelconque des revendications 31 à 57, dans lequel l'anticorps ainsi produit possède une affinité pour IgE qui est pratiquement identique ou supérieure à celle de MAE11 pour une IgE.
